# EUROPEAN PATENT SPECIFICATION

(11) **EP 1 876 962 B1**
(45) Date of publication and mention of the grant of the patent: **22.02.2017**
(21) Application number: 06750569.3
(22) Date of filing: 18.04.2006
(51) Int. Cl.: A61B 90/00, A61B 17/00, F16K 15/14, A61B 17/34, A61M 39/22, A61M 39/24, A61M 25/00

(54) **APPARATUS FOR SEALING A PUNCTURE IN TISSUE**
GERÄT ZUM VERSCHLUSS EINER PUNKTIONSSTELLE IN GEWEBE
APPAREIL PERMETTANT DE FERMER UN POINT DE PONCTION DANS DES TISSUS

(30) Priority: 22.04.2005 US 112877; 22.04.2005 US 112971
(43) Date of publication of application: 16.01.2008
(73) Proprietor: Access Closure, Inc., Santa Clara, CA 95054 (US)
(72) Inventor: BAGAOISAN, Celso, J., Union City, California 94587 (US); BLEAM, Jefferey, Boulder Creek, California 95006 (US); LEGUIDLEGUID, Roy, Union City, California 94587 (US); PAI, Suresh, S., Mountain View, California 94043 (US); DOMINGO, Juan, Union City, California 94587 (US)
(74) Representative: Prock, Thomas
(86) International application number: PCT/US2006/014562
(87) International publication number: WO 2006/115904

(56) References cited:
- WO-A-95/33510
- US-A- 4 598 707
- US-A- 4 948 092
- US-A- 5 085 249
- US-A1- 2004 249 342

## Description

### FIELD OF INVENTION

The present invention relates generally to apparatus and methods for sealing punctures in a body, to apparatus and methods for facilitating access through a puncture extending through tissue, and, more particularly, to apparatus and methods for deploying an occlusion element, such as a balloon or other expandable member, disposed on a guidewire or other flexible elongate member, to seal a puncture through tissue.

### BACKGROUND

Apparatus and methods are known for accessing a patient's vasculature percutaneously for performing a procedure within the vasculature. For example, a hollow needle may be inserted through a patient's skin and overlying tissue into a blood vessel. A guidewire is then passed through the needle into the blood vessel, whereupon the needle is removed. An introducer sheath is then advanced over the guidewire into the vessel, e.g., in conjunction with or subsequent to one or more dilators. A catheter or other device may be advanced through the introducer sheath and over the guidewire into a position for performing a medical procedure within the patient's body. In this manner, the introducer sheath facilitates introducing various instruments into the vessel, while minimizing trauma to the vessel wall and blood loss.

Upon completing the procedure, the instrument(s) and introducer sheath are removed, leaving a puncture extending between the skin and the vessel. To seal the puncture, external pressure may be applied to the overlying tissue, e.g., manually and/or using sandbags, until hemostasis occurs. This procedure, however, can be time consuming and expensive, requiring as much as an hour of a medical professional's time. It is also uncomfortable for the patient, and may require the patient to remain immobilized in an operating room, catheter lab, or holding area. In addition, a risk of hematoma exists from bleeding before hemostasis occurs.

Various apparatus and methods have been suggested for sealing a percutaneous puncture instead of or in addition to using external pressure. For example, U.S. Patent No. 5,108,421 to Fowler discloses using a collagen plug that is delivered into a puncture through tissue. After completing the procedure, the introducer sheath and/or guidewire used to access the patient's vasculature via the puncture are removed. In one embodiment, a catheter is inserted through the puncture into the blood vessel. A balloon on the catheter is expanded and then retracted until the balloon is disposed adjacent the puncture at the wall of the vessel. A plug is then advanced into the puncture until the plug contacts the balloon, thereby preventing the plug from entering the vessel. Once the plug is positioned within the puncture, the balloon is deflated and withdrawn, leaving the plug to expand and seal the puncture and/or promote hemostasis.

By way of another example, U.S. Patent Nos. 5,192,302 and 5,222,974 issued to Kensey et al. describe using a collagen plug that may be delivered through an introducer sheath into a puncture site.

Such sealing methods generally involve introducing plugs or other materials into the puncture after completing the procedure and removing the introducer sheath. With the introducer sheath removed, there is substantial risk of hematoma within the tissue surrounding the puncture as blood from the vessel leaks into the puncture, which may be uncomfortable and/or harmful to the patient. Further, temporary hemostasis devices for isolating the vessel from the puncture may be difficult to use effectively and/or may be expensive. Despite attempts to isolate the vessel from the puncture while delivering a plug or other sealing material, the sealing material may still leak and/or become exposed in the vessel, where the sealing material may risk causing an embolism in the vessel.

In US 2004/249342 A1 there is described an apparatus for providing hemostasis within a puncture extending through tissue according to the preamble of claim 1. This document US 2004/249342 A1 discloses a balloon catheter that includes a piston slidably received in a cylinder, thereby dividing the chamber into a proximal chamber and a distal chamber. To provide a biasing force, a compression spring or other mechanism may be provided in the proximal chamber of the housing, e.g., for biasing the piston away from the end wall. The cylinder and piston include marker bands such that, when the piston retracts within the cylinder, the piston marker may pass behind the cylinder marker and disappear to provide a visual indication.

WO 05/33510 A discloses a balloon catheter for angioplasty that includes an over-inflation device including a housing that includes an influent port, which can be connected to a syringe, an effluent port, which may be connected to the catheter, and a pressure release port that extends perpendicular to the influent and effluent ports. A movable wall is disposed within the housing for defining a variable volume pressure release chamber and a vent is provided in the housing for releasing inflation media to maintain a precalibrated maximum pressure.

US 4 948 092 discloses a combined check valve and relief valve assembly that includes a duckbill valve for preventing backflow.

It is against the background, and the limitations and problems associated therewith, that the present invention has been developed.

To achieve this, the apparatus for providing hemostasis within a puncture extending through tissue of the invention is characterized by the features claimed in the characterizing part of claim 1.

Advantageous embodiments of the invention are claimed in the subclaims.

The present invention is directed generally to apparatus and systems for facilitating access through a puncture through tissue, e.g., extending from a patient's skin to a blood vessel or other body lumen, and/or for sealing such punctures. More particularly, apparatus and systems are provided that include a guidewire or other elongate tubular member having a balloon or other expandable member thereon, and methods for using such tubular members are also provided.

In one embodiment, the expandable member may be expanded and/or collapsed by moving a piston within the tubular member to direct fluid into and/or out of the expandable member. In addition or alternatively, the expandable member may be inflated and/or deflated using a fluid dispensing device communicating with the lumen of the tubular member, e.g., that may be removably coupled to the tubular member.

In accordance with one embodiment, an occlusion member is provided for sealing a puncture. Generally, the occlusion member includes an elongate tubular member including a proximal end, a distal end, a lumen extending at least partially between the proximal and distal ends, and an expandable occlusion element disposed on a distal region of the tubular member. A piston may extend from and/or be movable within the lumen of the tubular member for directing fluid within the lumen into and/or out of the occlusion member such that movement of the piston relative to the tubular member may inflate and expand and/or deflate and collapse the occlusion element.

In another embodiment, a balloon wire device is provided for sealing a puncture through tissue. Generally, the device includes an elongate tubular or wire member including a proximal end, and a distal end sized and shaped for insertion into a puncture through tissue, e.g., having a profile similar to a conventional guidewire. The device includes a lumen extending axially through the wire member, and a balloon or other expandable member on a distal region of the wire member. The wire member may include one or more openings, e.g., in a proximal region of the wire member, that communicates with the lumen.

Optionally, the device may include a fluid dispensing device removably coupled to the wire member. When the fluid dispensing device is coupled to the device, fluid may be directed from the fluid dispensing device into lumen of the wire member via the opening(s) in the proximal end of the wire member. In one embodiment, the device may include a piston or other element movable within the wire member for facilitating delivery of the fluid and/or for expanding and/or collapsing the expandable member.

In another embodiment, a wire may be disposed inside the lumen having a first end affixed to the proximal end of the wire member and a second end affixed to the occlusion member. As the occlusion member is expanded, e.g., by delivering fluid into the occlusion member via the lumen of the wire member, the wire may be subjected to a compressive stress, causing the wire to buckle. Conversely, as the occlusion member is collapsed, e.g., by evacuating the fluid, the wire may extend axially to release the buckling stress, thereby extending the occlusion member as it collapses. In one embodiment, the wire may be formed from an elastic or superelastic material, allowing the wire to resiliently buckle and extend.

In yet another embodiment, a system is provided for introducing one or more instruments into a body lumen of a patient through a puncture extending from the patient's skin to the body lumen. Generally, the system includes a balloon wire or other occlusion member, such as those described above. In addition, the system may include a fluid dispensing device, an assembly for delivering a sealing compound into the puncture, and/or an introducer sheath.

The fluid dispensing device may be removably coupled to the occlusion member, e.g., for delivering fluid from the fluid dispensing device into a lumen of the occlusion member. The introducer sheath may include a proximal end, a distal end sized and shaped for insertion into the puncture, and a lumen extending between the proximal end and an opening in the distal end.

In accordance with another embodiment, a method is provided for sealing a puncture in a vessel using an occlusion member including an elongate tubular or wire member having a proximal end, a distal end, a lumen extending at least partially therebetween, and an expandable occlusion member on a distal region of the tubular member.

Generally, the distal end of the tubular member is introduced into a puncture through tissue with the occlusion member collapsed, e.g., until the occlusion member is disposed within a body lumen communicating with the puncture. A piston within the tubular member is moved to deliver fluid within the tubular member into the occlusion member, causing the occlusion member to expand. The tubular member may be at least partially retracted until the expanded occlusion member substantially seals the body lumen from the puncture.

In another embodiment, a method is provided for sealing a puncture in a wall of a blood vessel or other body lumen using an occlusion member including an elongate tubular member and an expandable occlusion member on a distal region of the tubular member. A fluid dispensing device may be coupled or otherwise provided on a proximal end of the tubular member. The distal end of the tubular member may be introduced through the puncture with the occlusion member collapsed. Fluid may be dispensed into the tubular member from the fluid dispensing device to expand the occlusion member. The expanded occlusion member may be retracted against the wall of the body lumen to substantially seal the puncture.

In accordance with still another embodiment, a method is provided for preparing an occlusion member, the occlusion member comprising an elongate tubular member including a proximal end, a distal end having an inflatable occlusion element thereon, a lumen extending between the proximal and distal ends communicating with an interior of the occlusion element, and an opening at an intermediate location on the tubular member communicating with the lumen. In one embodiment, the method includes advancing a distal end of an elongate member into the proximal end of the tubular member until the distal end of the elongate member is disposed proximal to the opening and a proximal end of the elongate member extends proximally from the tubular member; evacuating air from within the lumen and the interior of the occlusion member via the opening; delivering a substantially incompressible inflation media into the lumen without substantially expanding the occlusion element; and advancing the distal end of the elongate member distally beyond the opening to substantially isolate the lumen with the fluid therein without substantially expanding the occlusion element. The method may further include advancing the elongate member distally within the tubular member to expand the occlusion element.

In accordance with yet another embodiment, an apparatus is provided for providing hemostasis within a puncture extending through tissue. The apparatus includes an elongate tubular member having proximal and distal ends defining a longitudinal axis therebetween, and a lumen extending between the proximal and distal ends. The elongate member may include an expandable member, e.g., a balloon, disposed on or adjacent to the distal end, and a hub on the proximal end that includes an interior communicating with the lumen of the elongate member. The hub may include a valve communicating with the interior, and a plunger having a fluid port thereon that is movable into and out of the valve.

For example, in one embodiment, the valve may be a one-way valve allowing fluid to be delivered into the interior of the hub. When fluid is delivered into the interior, the fluid may travel through the lumen to expand the expandable member. The plunger may be advanceable into the valve to open the valve and allow fluid to be evacuated from the interior. Optionally, the valve may automatically close when the plunger is withdrawn to substantially seal any remaining fluid within the interior. In addition or alternatively, the plunger may be biased such that when the plunger is released, it automatically withdraws from the valve, allowing the valve to close again to its one-way configuration.

The apparatus may also include a piston movable within the interior, and a pressure indicator coupled to the piston. The pressure indicator may be used to determine when the expandable member is expanded to a predetermined size and/or shape, e.g., when a predetermined volume of fluid is directed into the interior. For example, when fluid is delivered into the interior through the valve, the piston may move proximally until the pressure indicator is visible, indicating a desired volume of fluid has been delivered into the interior to expand the expandable member. In one embodiment, the piston may be coupled to a distal end of the expandable member such that the piston moves proximally as fluid is directed into the interior, thereby shortening the expandable member as it expands.

Optionally, the hub may include a pressure relief feature to avoid over-pressurization, e.g., to prevent the expandable member from expanding beyond a desired size and/or shape. In one embodiment, the pressure relief feature may be an evacuation port adjacent the piston that communicates with the interior of the hub when the piston moves to a predetermined proximal position, corresponding to a predetermined pressure and/or volume of the fluid within the interior. The evacuation port may communicate with a reservoir or vent to atmosphere, thereby allowing excess fluid to be evacuated from the interior when the predetermined pressure is exceeded. Alternatively, the pressure relief feature may be a valve that opens when a predetermined pressure within the interior is exceeded, allowing fluid within the interior to leak or otherwise escape from the hub.

In another embodiment, an apparatus for providing hemostasis within a puncture through tissue includes an elongate tubular member having proximal and distal ends defining a longitudinal axis therebetween, and a lumen extending between the proximal and distal ends. The elongate member has an expandable member disposed at or adjacent to the distal end that is expandable between collapsed and enlarged conditions, and a hub on the proximal end. The hub may include an interior communicating with the lumen of the elongate member, and a piston slidable within the interior.

The piston may be coupled to the expandable member and may be biased to a distal position within the hub to extend the expandable member in the collapsed condition. The hub may include a valve for receiving a source of fluid therethrough, e.g., a fluid port coupled to a plunger, to deliver or aspirate fluid from the interior. When fluid is delivered into the interior and the lumen to expand the expandable member, the piston may be directed proximally against the bias, thereby shortening the expandable position as it expands. A pressure indicator may be coupled to the piston that provides a visual or other indicator when sufficient fluid is delivered to expand the expandable member to a desired size and/or shape. When fluid is evacuated from the interior via the valve, the piston may resiliently return towards the distal position, thereby extending the expandable member as it collapses.

Optionally, the hub may include a pressure-relief feature and/or a one-way valve-plunger mechanism. In addition or alternatively, the apparatus may include an introducer or other sheath, including proximal and distal ends, and a sheath lumen extending therebetween sized for receiving the elongate member therein when the expandable member is collapsed. In addition, a source of sealing compound may be provided that may be coupled to the proximal end of the sheath for delivering a sealing compound into the sheath lumen through the tubular member. In exemplary embodiments, the source of sealing compound may include multiple polymers that may be mixed and/or otherwise injected as a liquid, or a solid plug or carrier.

In accordance with another embodiment, a method is provided for providing hemostasis of a puncture, e.g., extending through tissue and/or communicating with a body lumen. In an exemplary embodiment, the body lumen may be a blood vessel, e.g., a femoral, carotid, or other peripheral artery. Initially, an apparatus may be provided that includes an elongate tubular member having a distal end carrying an expandable member, and a proximal end including a hub.

The distal end of the tubular member may be introduced into the puncture until the expandable member is disposed within the body lumen. Fluid may be introduced into the hub through a one-way valve. As fluid enters the hub, the fluid may pass through the tubular member to expand the expandable member within the body lumen. In one embodiment, fluid delivery may continue until a pressure indicator on the hub indicates that the expandable member has been expanded to a desired size and/or shape. Optionally, fluid delivery may cause the expandable member to shorten as it expands.

With the expandable member expanded, the apparatus may be at least partially withdrawn from the puncture until the expandable member contacts a wall of the body lumen, thereby substantially sealing the puncture from the body lumen. Optionally, a sealing material, e.g., a multiple component liquid sealing compound and/or a solid plug or carrier, may be introduced into the puncture around the tubular member to enhance hemostasis.

Fluid may be evacuated from the expandable member by overcoming the one-way nature of the valve, e.g., by directing a nipple, plunger, or other element into the valve. For example, a plunger may be advanced through the valve such that fluid in the hub escapes through the valve or is aspirated into a syringe or other source of vacuum. As fluid is evacuated from the hub, the expandable member may collapse and/or extend. With the expandable member collapsed, the apparatus may be withdrawn completely from the puncture, e.g., through the sealing material if such material is delivered into the puncture.

Optionally, pressure maybe applied to skin overlying the body lumen, e.g., to at least partially suppress fluid flow through the body lumen as the apparatus is withdrawn from the puncture.

In one embodiment, before using the apparatus, an introducer or other sheath may be introduced through the puncture into the body lumen, and the apparatus may be introduced into the puncture through the introducer. With the apparatus sealing the puncture from the body lumen, sealing material maybe introduced into the puncture, e.g., through and/or around the introducer. While or after delivering the sealing material into the puncture, the introducer may be withdrawn from the puncture, e.g., before or after removing the apparatus.

Other aspects and features of the invention will become apparent from consideration of the following description taken in conjunction with the accompanying drawings.

### BRIEF DESCRIPTION OF THE DRAWINGS

The drawings illustrate exemplary embodiments of the invention, in which:
FIG. 1 is a side view of an exemplary embodiment of an apparatus for temporary hemostasis, including a balloon on a distal end and a hub on a proximal end of the apparatus with a one-way valve for delivering fluid into the hub to expand the balloon.
FIG. 2 is a cross-sectional side view of the hub of the apparatus of FIG. 1, showing a button shown in an "up" position, which may be used to open the one-way valve.
FIG. 3 is a cross-sectional side view of the hub of FIG. 2 with the button in a "down" position, allowing fluid to be evacuated from within the hub.
FIG. 4 is a cross-sectional side view of the hub of FIG. 2, illustrating fluid being delivered from a syringe into the hub with the button in the "up" position. As fluid is introduced into the hub, a pressure indicator may project proximally from the hub.
FIG. 5 is a cross-sectional side view of the balloon of FIG. 1 being expanded as fluid is delivered into the hub. The balloon may be expanded to a desired size when the pressure indicator projects from the hub, as shown in FIG. 4.
FIG. 6 is a cross-sectional side view of the hub of FIG. 2, showing an evacuation port communicating with an interior of the hub through the valve, allowing fluid to escape from the interior to prevent over-inflation of the balloon.
FIG. 7 is a cross-sectional side view of the balloon of FIG. 1 being collapsed as fluid is evacuated from the hub.
FIGS. 8A-8F are cross-sectional views of a puncture extending through tissue to a body lumen, showing a method for sealing the puncture.
FIG. 9 is a cross-sectional view of a puncture extending through tissue to a body lumen, showing a method for sealing the puncture.
FIG. 10 is a side view of a system for sealing a puncture, including an introducer sheath, an occlusion member, a delivery sheath, and a syringe assembly for delivering sealing compound via the delivery sheath.
FIGS. 11A and 11B are cross-sectional side views of the occlusion member of FIG. 10, showing a method for assembling the occlusion member.
FIGS. 11C and 11D are cross-sectional details of the occlusion member of FIG. 10, showing a fluid dispensing device for delivering fluid into a wire member of the occlusion member.
FIGS. 12A-12C are cross-sectional views of a patient's body, illustrating a method for delivering a sealing compound a puncture extending between the patient's skin and a blood vessel.
FIGS. 13A-13C are cross-sectional views of a patient's body, showing a method for delivering a sleeve and introducer sheath into the puncture of FIGS. 12A-12C after delivering a sealing compound therein.
FIGS. 14A and 14B are cross-sectional side views of another embodiment of an occlusion member including a balloon in an expanded (i.e., deployed) state and a collapsed (i.e., un-deployed) state, respectively.
FIG. 15 is a cross-sectional side view of a fluid dispensing device and associated syringe for inflating and/or deflating the balloon of FIGS. 14A and 14B.

### DETAILED DESCRIPTION OF THE ILLUSTRATED EMBODIMENTS

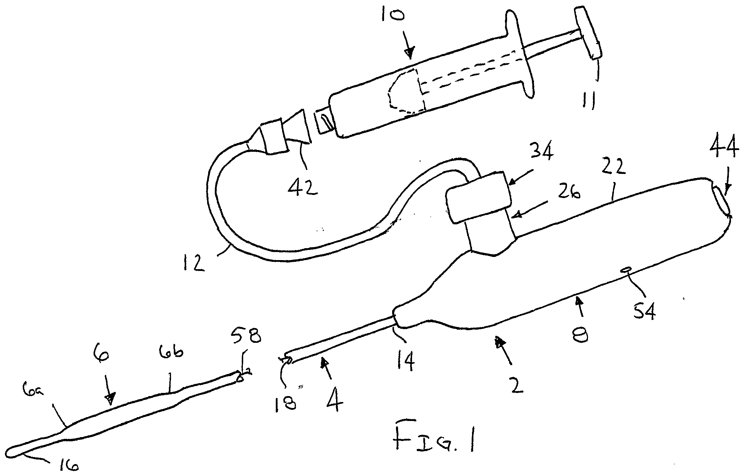

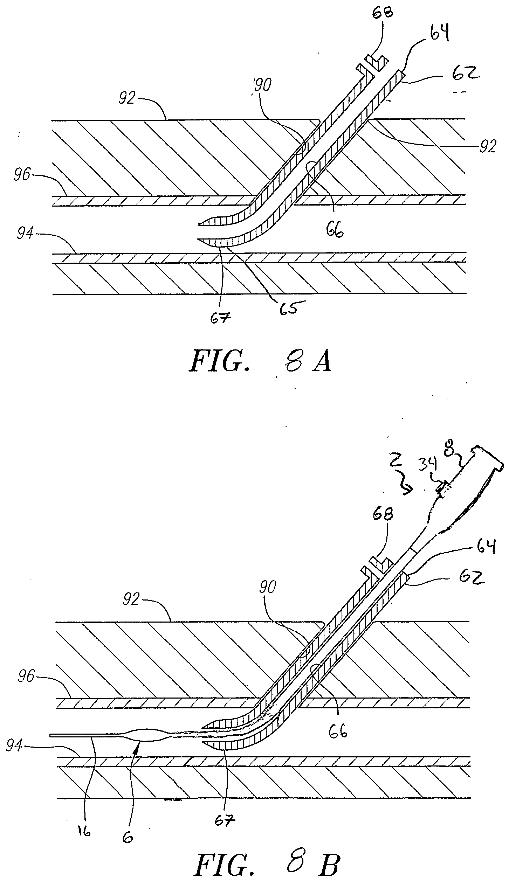

Turning to the drawings, FIGS. 1-7 show an exemplary embodiment of an apparatus 2 for providing temporary hemostasis within a puncture extending through tissue (not shown, see, e.g., FIGS. 8A-8F). Generally, the apparatus 2 includes an elongate member 4 having a balloon or other expandable member 6, a hub subassembly 8, and a source of inflation media, e.g., syringe 10, coupled to the hub subassembly 8, e.g., via a conduit 12, such as flexible tubing and the like.

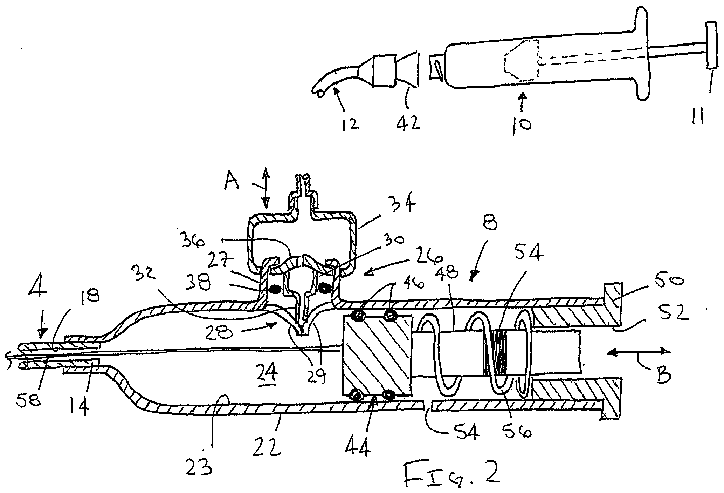

With particular reference to FIGS. 1 and 2, the elongate member 4 may be an elongate tubular body including a proximal end 14, a distal end 16, and a lumen 18 extending therebetween, thereby defining a longitudinal axis 20. The elongate member 4 may be flexible, semi-rigid, or rigid, e.g., having a uniform or variable flexibility along its length. The elongate member 4 may be formed from a variety of materials providing a desired rigidity, e.g., plastic, such as polyamide, PEEK, nylon, PET, PEBAX, polyethylene, and/or metal, such as stainless steel or a nickel-titanium alloy, and may be fabricated using known processes, e.g., extrusion, roll forming, machining, and the like. Optionally, a lubricious coating (not shown) may be provided on an exterior of the elongate member 12, e.g., DOW 360 silicone fluid.

In one embodiment, the distal end 16 may be substantially flexible such that the distal end 16 may curve, bend, or otherwise conform substantially to the contour of a puncture and/or body lumen (not shown) into which the distal end 16 is advanced. The distal end 16 of the elongate member 4 may have a size sufficient to be inserted into a relatively small puncture and/or body lumen. For example, the distal end 16 (and possibly the remainder of the elongate member 4) may have an outer diameter between about 0.010-0.030 inch (0.25-0.75 mm), or less than about 0.020 inch (0.5 mm).

Still referring to FIGS. 1 and 2, the hub subassembly 8 generally includes a housing 22 affixed to the proximal end 14 of the elongate member 4. The housing 22 includes an interior or cavity 24 that communicates with the lumen 18 of the elongate member 4, and a valve assembly 26 for accessing the interior 24. In one embodiment, the valve assembly 26 may include a one-way valve, such as a duckbill valve 28. The duckbill valve 28 may include mating flaps 29 that maybe selectively opened to allow communication with the interior 24. For example, the valve 28 may be configured such that the flaps 29 open automatically when the pressure is greater within the valve assembly 26 than within the interior 24, but otherwise are biased to close together to provide a substantially fluid-tight seal.

The valve assembly 26 may also include a plunger or nipple 30 having an outer cap 36 and a fluid port 32 extending from the cap 36. The nipple 30 may be movable within valve housing 27 in a direction represented by arrow A shown in FIG. 2, e.g., to direct the nipple 30 between the flaps 29 to open or otherwise overcome the bias of the valve 28, as explained further below. Optionally, a cover or button 34 may be provided over the valve assembly 26 that may be coupled to the cap 36, e.g., to facilitate directing the nipple 30 downwardly or otherwise into the valve 28.

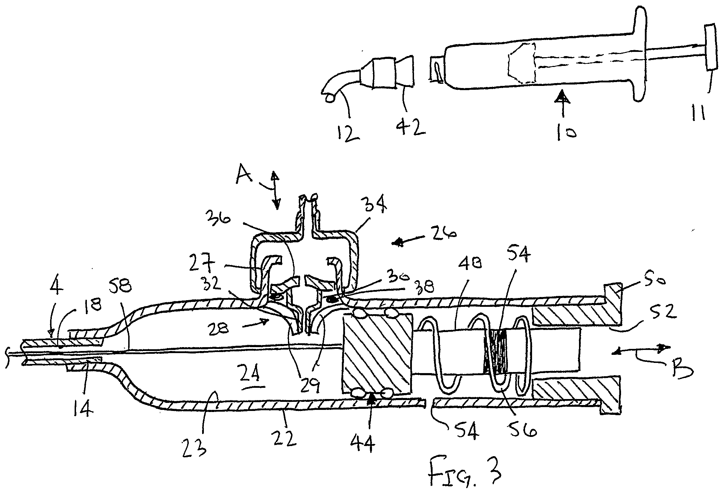

Still referring to FIG. 2, the nipple 30 may be moved by depressing the button 34 or cap 36. The button 34 may be able to toggle between "up" and "down" positions, as shown in FIGS. 2 and 3, respectively. In one embodiment, the button 34 may be biased towards the "up" position, e.g., by a spring or other biasing device (not shown) disposed within the valve assembly 26, for example, between the button 36 and the flaps 29. Alternatively, the button 34 may be releasably securable in the down position using any number of known mechanisms, e.g., one or more detents, latches, and the like, such that the button 34 may be alternately securable in the "up" and "down" positions. The nipple 30 may be sealed in the valve assembly 26, e.g., using an o-ring 38 or similar seal, that provides a substantially fluid-tight seal, yet allows the nipple 30 to move between the "up" and "down" positions.

The nipple 30 may communicate through the valve housing 27 and/or button 34 with a conduit, such as flexible tubing 12 and the like, that may terminate in a connector 42, such as a male or female Luer lock connector 42. The connector 42 may be configured to connect or otherwise mate with a syringe 10 or other source of inflation media, e.g., to allow fluid infusion/evacuation, as described further elsewhere herein.

Still referring to FIG. 2, the hub subassembly 8 includes a piston 44 slidable within the housing 22, e.g., axially in a direction represented by arrow B in FIG. 2. The piston 44 may include a seal 46, for example, one or more o-rings and the like, that may form a substantially fluid-tight seal between the piston 44 and an inner surface 23 of the housing 22, yet allow the piston 44 to move axially within the housing 22. Consequently, fluid may be prevented from passing proximally past the seal 46 between the piston 44 and the housing 22.

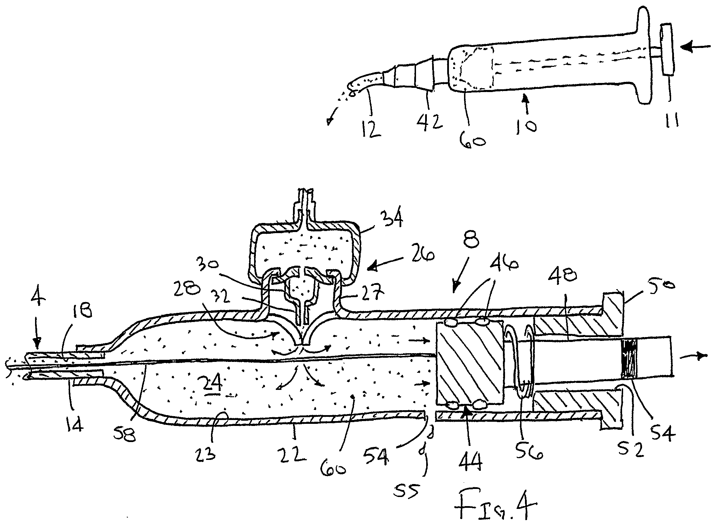

Optionally, the hub subassembly 8 may include a pressure relief feature 54 that may cooperate with the piston 44 to prevent over-pressurization, as explained further below. For example, as shown, the pressure relief feature 54 may be a side port or other opening in the housing 22 adjacent the piston 44. When the piston 44 is in a distal position, such as that shown in FIGS. 2 and 3, the pressure relief feature 54 may be substantially isolated from the interior 24. When the piston 44 moves proximally to a proximal position, such as that shown in FIG. 4, the pressure relief feature 54 may communicate with the interior 24, as explained further below. In an alterative embodiment, the pressure relief feature may be a relief valve (not shown) in the hub subassembly 8 adjacent the interior 24 that may open when a predetermined pressure is exceeded.

A pressure indicator may be provided on or otherwise coupled to the piston 44. In one embodiment, the piston 44 may include a shaft or member 48 that projects proximally from the piston 44 and carries a visual or other indicator 54. For example, as shown in FIGS. 2-4 and 6, the pressure indicator 54 may be a colored band around the shaft 48 or other proximal portion of the piston 44. Optionally, other pressure indicators may be provided that indicate when the piston 44 has moved to a predetermined proximal position within the housing 22, e.g., cooperating detents that "click" or provide other audible and/or tactile feedback to the user.

With reference still to FIG. 2, the hub subassembly 8 may also include an abutment or proximal end 50 having an orifice 52 sized to permit the shaft 48 of the piston 44 to pass therethrough, e.g., to expose the pressure indicator 48, as shown in FIG. 4. In this regard, the pressure indicator 48 maybe able to move proximally and project outwardly from the proximal end 50 of the hub subassembly 8. In the embodiment shown, the pressure indicator 48 includes a visual indicator 54, e.g., a color band surrounding all or a part of the pressure indicator 48. The visual indicator 54 may provide a visual indication that a predetermined pressure and/or volume of fluid has been introduced into the hub subassembly 8, e.g., to expand the balloon 6 to a desired size and/or shape.

For example, the predetermined pressure may correspond to a desired pressure for the balloon 6, e.g., to ensure that the balloon 6 is expanded to a desired diameter and/or to prevent risk of the balloon 6 rupturing. Alternatively, the visual indicator 54 may include a series of graduations or other indicia (not shown) that may be used to ascertain the degree of expansion of the balloon 6.

Optionally, the piston 44 may be biased, e.g., towards the proximal or distal position. For example, as seen in FIG. 2, an extension spring 56 or other biasing member may be coupled between the piston 44 and at the other end to the abutment 50. The spring 56 may bias the piston 44 towards the distal position, i.e., tending to move the piston 44 distally absent any pressure from fluid in the interior 24 of the housing 22.

In one embodiment, the piston 44 may be coupled to the expandable member 4 to shorten or extend the balloon 6 as the piston moves relative to the hub subassembly 8. For example, a wire, filament, or other connector member 58 may be secured at one end to the piston 44 and at its other end to a distal end 6a of the balloon 6 or distally beyond the balloon 6. When fluid is introduced into the interior 24 of the hub subassembly 8, the piston 44 may move proximally, e.g., toward the hub subassembly abutment 50 against the bias of the spring 56. Movement of the piston 44 proximally thereby applies tension or otherwise pulls the connector member 58 proximally, which shortens the balloon 6, e.g., to facilitate creating a toroidal shape when the balloon 6 is expanded.

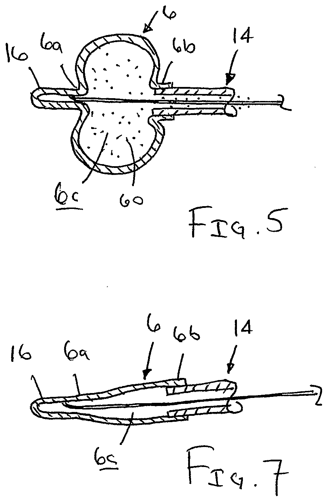

With reference to FIGS. 1, 5, and 7, the balloon 6 may be an expandable body expandable between a contracted condition (such as that shown in FIGS. 1 and 7) and an enlarged condition (such as that shown in FIG. 5). The balloon 6 maybe formed from a flexible, substantially inelastic material, e.g., a nonelastomeric material, such as PET, nylon, polyethylene, polyurethane, PEBAX, and the like. For example, the balloon 6 may be a substantially noncompliant body that may expand to a predetermined size once a minimum pressure is introduced into its interior. In this embodiment, the size of the balloon 6 in the enlarged condition may be substantially fixed. Alternatively, the balloon 6 may be formed from an elastic material, such that the size in the enlarged condition is dependent upon the pressure or volume of fluid delivered within the balloon 6, as is known in the art.

As best seen in FIGS. 1 and 5, the distal end 6a of the balloon 6 may be extended distally to provide a floppy or otherwise substantially atraumatic tip for the apparatus 2. In an exemplary embodiment, the distal end 6a of the balloon 6 may have a length of at least about fifty millimeters (50 mm).

In the contracted condition, the balloon 6 may conform substantially to the diameter of the elongate member 4. In one embodiment, tension may be applied between the proximal and distal ends of the balloon 6 by the connector member 58 such that the balloon 6 is under slight axial tension in the contracted condition, which may minimize risk of the balloon 6 expanding, kinking, otherwise increasing in cross-section and/or catching on anything contacted by the balloon 6.

Turning to FIGS. 4 and 7, the balloon 6 maybe expanded to the enlarged condition by introducing inflation media 60 into the interior 24 of the housing 22, through the lumen 18 of the elongate member 4, and, consequently, into the interior 6c of the balloon 6. The inflation media 60 may be a substantially incompressible fluid, e.g., saline, or a compressible fluid, such as carbon dioxide, nitrogen, or air. As fluid 60 is introduced into the interior 24 of the housing 22, the fluid pressure within the interior 24 may exceed the bias of the spring 56, causing the piston 44 to move proximally within the housing 22, thereby pulling the wire 58 and shortening the balloon 6 as it expands.

Additional information on possible construction of the expandable member 6 or other components of the apparatus 2 may be found, for example, in co-pending application Serial Nos. 10/454,362, filed June 4, 2003, and 10/806,952, filed March 22, 2004.

Optionally, as shown in FIGS. 8A-8F, the apparatus 2 may include other components, e.g., to provide a kit for performing a procedure on a patient. For example, an introducer or other access or delivery sheath 62 may be provided that includes a proximal end 64, a distal end 65, and a lumen 66 extending therebetween. The introducer sheath 62 may include a tapered distal tip 67, e.g., for facilitating advancing the introducer sheath 62 through a puncture, as is known in the art.

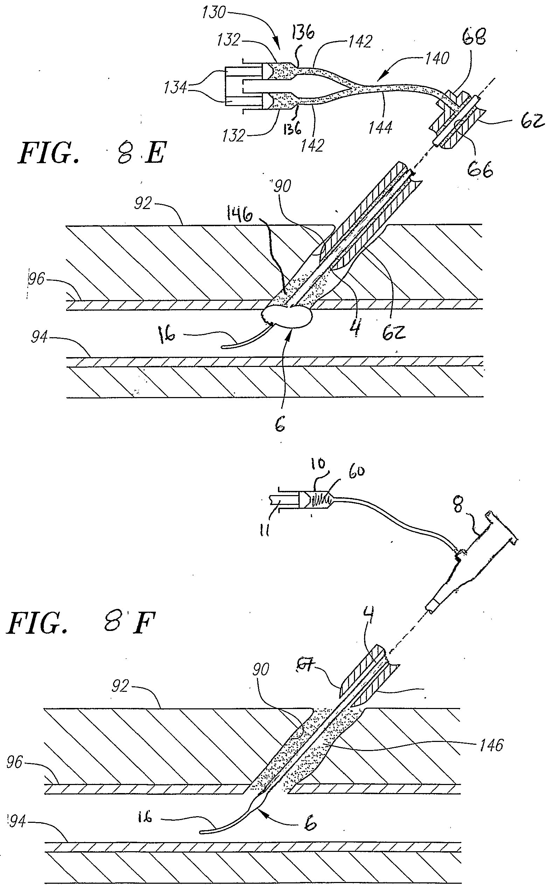

In addition, the introducer sheath 62 may include a side port 68 on the proximal end 64 communicating with the lumen 66 and/or may include one or more seals (not shown), e.g., to prevent substantial proximal flow of fluid through the lumen 66, also as is known in the art. As shown in FIG. 8E, a source of sealing compound 130 may be connectable to the side port 68, e.g., for delivering a sealing compound 146 into the lumen 66 of the introducer sheath 62.

With continued reference to FIG. 8E, a dual syringe assembly 130 may be provided that includes two components of a sealing compound. In an exemplary embodiment, a polymer precursor may be provided in each syringe 132 of the syringe assembly 130. A "Y" fitting 140 maybe provided that includes proximal sections 142 that communicate with a single distal section 144. The proximal and distal sections 142, 144 may include connectors, e.g., luer lock connectors and the like (not shown), for connecting with outlets 136 of the syringes 132 and with the side port 68 of the introducer sheath 62. Thus, the "Y" fitting 140 may be connectable to outlets 136 of the syringes 132 such that the components ejected out of the syringes 132 may mix before being delivered into the side port 68 of the introducer sheath 62. The "Y" fitting 140 may include one or more components, e.g., separate lengths of tubing and the like (not shown), as will be appreciated by those skilled in the art.

In one embodiment, the components are polymer precursors that mix to create a hydrogel. Additional information on hydrogels and systems and methods for delivering them may be found in U.S. Patent Nos. 6,152,943, 6,165,201, 6,179,862, 6,514,534, and 6,379,373, and in co-pending applications Serial Nos. 09/776,120 filed February 2, 2001, 10/010,715 filed November 9, 2001, and 10/068,807 filed February 5, 2002.

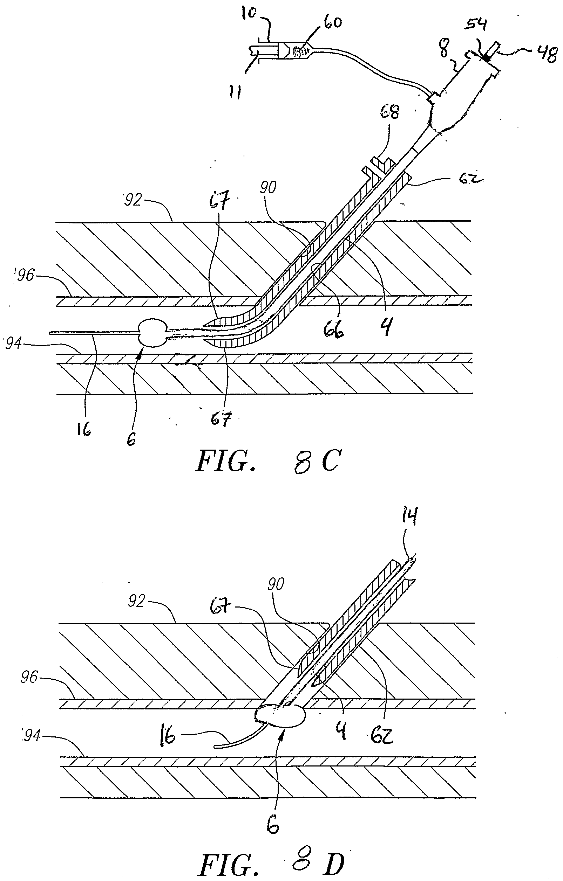

In addition, the kit may include a syringe 10 (as shown in FIGS. 8C and 8F) or other source of inflation media 60 that may be coupled to the hub subassembly 8, as explained above. Optionally, the kit may also include a stylet or obturator (not shown) that maybe inserted into the lumen 66 of the introducer sheath 62, e.g., to facilitate percutaneously inserting the introducer sheath 62 through tissue. In addition or alternatively, one or more guidewires (not shown) may also be provided.

Turning to FIGS. 8A-8F, an exemplary method for sealing a passage through tissue is shown. In an exemplary embodiment, the passage is a percutaneous puncture 90 extending from a patient's skin 92 through tissue to a blood vessel or other body lumen 94. For example, the vessel 94 maybe a peripheral artery, e.g., a femoral artery, a carotid artery, radial artery, and the like.

Initially, as shown in FIG. 8A, an introducer sheath 62 may be placed within the puncture 90 such that the distal tip 67 is disposed within the vessel 94. For example, a stylet having a sharpened distal tip (not shown) may be inserted through the lumen 66 of the introducer sheath 62 such that the sharpened distal tip extends beyond the distal tip 67 of the introducer sheath 62. The introducer sheath 62 and stylet may then be inserted directly through the patient's skin 92 until the distal tip 67 is disposed within the vessel 94. Alternatively, the introducer sheath 62 may be advanced over a guidewire previously inserted through the puncture 90 into the vessel 94, using known procedures.

One or more instruments (not shown) may be advanced through the introducer sheath 62 and into the vessel 94, e.g., to perform one or more diagnostic and/or therapeutic procedures within the patient's body. The one or more instruments may include catheters, e.g., balloon catheters, stent delivery catheters, imaging catheters, and the like, guidewires, and/or other devices. Upon completing the procedure(s), any instruments may be removed and the puncture 90 maybe sealed using an apparatus, such as that shown in FIGS. 1-7 and described above.

For example, turning to FIG. 8B, with the balloon 6 in the contracted condition, the distal end 16 of the apparatus 2 may be inserted through the lumen 66 of the introducer sheath 62 until the balloon 6 is disposed within the vessel 94. Optionally, the apparatus 2 may include one or more markers, e.g., radiopaque markers (not shown), to facilitate monitoring insertion of the apparatus 2 using external imaging, e.g., fluoroscopy, ultrasound, magnetic resonance imaging ("MRI"), and the like.

Alternatively or in addition, one or more visual markers (not shown) may be provided, e.g., on the proximal end 14 of the elongate member 4. The markers may include one or more colored bands at predetermined locations along a length of the elongate member 4 relative to the balloon 6. For example, a distance between a band on the proximal end 14 of the elongate member 4 may correspond to a length of the introducer sheath 62, thereby providing a visual indication when the apparatus 2 has been advanced sufficiently to expose the balloon 6 beyond the distal tip 67 of the introducer sheath 62.

Optionally, with additional reference to FIGS. 1-6, the balloon 6 and/or apparatus 2 may be prepared before insertion into the introducer sheath 62 and/or puncture 90, e.g., to collapse the balloon 6. For example, syringe 10 (or other source of vacuum) may be secured to the connector 42 on the end of the conduit 12. The button 34 may be depressed, thereby pushing down the nipple 30 such that the fluid port 32 is exposed to the interior 24 of the housing 22, as shown in FIG. 3. With the nipple 30 advanced through the valve 28, the valve 28 may be opened, allowing fluid within the interior 24 of the housing 22 to be evacuated. For example, plunger 11 on the syringe 10 may be pulled proximally to draw a vacuum in the syringe 10, thereby evacuating any air or other fluid within the interior 24 of the housing 22. Consequently, any residual air or other fluid within the balloon 6 may also by evacuated, enhancing collapsing the balloon 6.

The button 34 may then be released or otherwise returned to the "up" position, shown in FIG. 2, retracting the nipple 30 and fluid port 32 out of the valve 28, thereby substantially sealing the fluid path to the balloon 6 from ambient pressure outside apparatus 2. This preparation, i.e., subjecting the balloon 6 to a vacuum before insertion, may minimize the profile of the balloon 6 in the contracted condition, which may facilitate inserting the apparatus 2 into the introducer sheath 62 and/or puncture 90.

Turning to FIG. 8C, once the collapsed balloon 6 is advanced through the introducer sheath 62 into the vessel 94, the balloon 6 may be expanded to the enlarged condition. For example, a source of inflation media may be connected to connector 42 of the hub subassembly 8. Alternatively, other sources of inflation media may be attached to the connector 42 on the conduit 12. The syringe 10 may be the same or different from the syringe 10 used to evacuate the fluid path to the balloon 6.

As shown in FIG. 4, plunger 11 of syringe 10 may be advanced distally, forcing inflation media 60 into the internal 24 of the housing 22. This causes the inflation media 60 to pass through the valve assembly 26, opening the flaps 29 of the valve 28, allowing the inflation media 60 to enter the interior 24 of the housing 22. The inflation media 60 may travel along the fluid path to the balloon 6, i.e., into the interior 24, through the lumen 18, and into the interior 6c of the balloon 6, as shown in FIG. 5.

As the plunger 11 of the syringe 10 is depressed, fluid pressure builds within the interior 24 of the housing 22 and lumen 18, causing the balloon 6 to inflate to the enlarged condition. Simultaneously, the increasing fluid pressure may also cause the piston 44 (and pressure indicator 48) to move proximally, as shown in FIG. 4. In one embodiment, the plunger 11 is depressed until the visual indicator 54 projects from the abutment 50 of the hub subassembly 8. When the visual indicator 54 is in this position, the user may be informed that a desired pressure or volume has been achieved within the balloon 6, which may correspond to a desired size and/or shape for the balloon 6, such as that shown in FIG. 5.

If additional fluid is delivered into the hub subassembly 8 after the desired volume and/or pressure is achieved, the hub subassembly 8 may include a pressure relief feature to prevent over-inflation of the balloon 6, which may otherwise damage or even break the balloon 6. For example, the pressure relief feature may be a side port 54 located in the housing 22 at a location adjacent the piston 44. When the piston 44 is directed proximally such that the visual indicator 54 appears, the side port 54 may remain substantially isolated from the interior 24 of the housing 22.

If additional fluid is delivered, the piston 44 may move proximally beyond this position, thereby exposing the interior 24 of the housing 22 to the side port 54. This exposure causes any excess fluid (represented by drops 55) from escaping through the side port 54 until the piston 44 returns to the proximal position where the side port 54 is no longer exposed to the interior 24. Thus, the side port 54 may provide a safety feature, allowing only a predetermined pressure and/or volume being present within the interior 24 of the housing 22. As shown in FIG. 5, in the enlarged condition, the balloon 6 may be expanded to a desired diameter and shortened to provide a toroidal shape.

Turning to FIG. 8D (which omits the proximal components of the apparatus 2 merely for simplicity), the apparatus 2 may be partially withdrawn from the puncture 90 with the balloon 6 in the enlarged condition, i.e., until the balloon 6 engages the puncture 90. The balloon 6 may substantially seal the puncture 90, i.e., substantially isolating the puncture 90 from the interior of the vessel 94. Thus, the apparatus 2 may provide temporary hemostasis, e.g., preventing blood from passing through the puncture 90. Thus, even without the additional steps that follow, the apparatus 2 may be used to provide hemostasis in emergency situations in order to minimize loss of blood until a puncture victim may be treated.

As explained above, the balloon 6 may at least partially evert in the enlarged condition, which may provide hemostasis, while still allowing blood flow to continue along the vessel 94. For example, as shown in FIG. 8D, the diameter of the balloon 6 may be substantially greater than its length in the enlarged condition. Thus, when the balloon 6 is pulled into engagement with the wall 96 of the vessel 94, at least a portion of the vessel 94 lumen may remain unobstructed, as shown.

Optionally, in order to maintain the balloon 6 substantially against the puncture 90 without requiring an individual to hold the apparatus 2, a tensioner or other external device (not shown) may be provided that may apply a proximal force to the apparatus 2 to maintain the balloon 6 substantially against the puncture 90, as disclosed in application Serial Nos. 10/454,362 and 10/806,952.

Optionally, turning to FIG. 8E, a sealing compound 146 maybe delivered into the puncture 90. For example, the sealing compound 146 maybe a liquid or other flowable material that may be introduced into the puncture 90. Because of the hemostasis provided by the balloon 6, the sealing compound 146 may be delivered without substantial concern that the sealing compound 146 may leak into the vessel 94.

In an exemplary embodiment, the sealing compound may include multiple component polymer precursors that create a hydrogel when mixed together. Such a sealing compound may be particularly useful, because it may be substantially harmless to the patient even if it somehow leaks into the vessel 94. In fact, such polymer precursors, if leaked into a vessel, may simply dilute and flow away, where they may be metabolized naturally without substantial risk of creating thrombus. This is another reason why it may be useful to seal the puncture 90 with an everted balloon 6, while still allowing fluid to continue to flow along the vessel 94, as described above. If the hydrogel leaks into the vessel 94 around the balloon 6, blood flow may dilute and carry the hydrogel away, where it may be safely metabolized naturally, e.g., by the liver.

As shown in FIG. 8E, a two-part sealing compound 146 is shown contained within a dual syringe assembly 130. The polymer precursors or other components in the syringes 132 may be mixed or otherwise prepared using known procedures. The plungers 134 of the syringes 132 may be linked such that they may be depressed substantially simultaneously, thereby delivering the precursors simultaneously. The precursors may mix in the "Y" fitting 140 into a liquid sealing compound 146, and then be delivered into the side port 68 of the introducer sheath 62. Alternatively, an auto injector device, including a spring, motor, pneumatic pressure, and the like (not shown) may be provided for delivering the precursors at a desired substantially continuous rate, as disclosed in application Serial No. 10/806,952. Such a device may prevent unintended pauses during delivery, which may cause the "Y" fitting 140 or other passages through which the sealing compound passes from becoming obstructed.

The liquid sealing compound 146 may be delivered through the lumen 66 of the introducer sheath 62 out the distal tip 67 into the puncture 90. The introducer sheath 62 may remain stationary as the sealing compound 146 is delivered, thereby allowing the sealing compound to flow into the puncture 90 around the introducer sheath 62. Alternatively, the introducer sheath 62 may be withdrawn proximally from the puncture 90 as the sealing compound 146 is delivered, thereby filling the puncture tract with the sealing compound 146, as shown in FIG. 8F. Optionally, other components (not shown) may be provided on the apparatus 2 and/or introducer sheath 62 to enhance delivery of the sealing compound, as disclosed in application Serial No. 10/454,362.

It will be appreciated that other devices may be used for delivering sealing material or even a sealing device into the puncture 90. For example, other apparatus for delivering liquid sealing compounds, including single or multiple lumens (not shown), may be advanced over the apparatus 2, e.g., through the introducer sheath 62. Alternatively, the introducer sheath 62 may be removed, before such delivery apparatus are advanced over the apparatus 2 into the puncture 90. In a further alternative, a solid plug may be advanced into the puncture 90 adjacent or around the apparatus 2. Thus, the balloon 6 may provide hemostasis and/or prevent a plug or other solid component or liquid sealing compound 146 from entering the vessel 94 as it is introduced into the puncture 90.

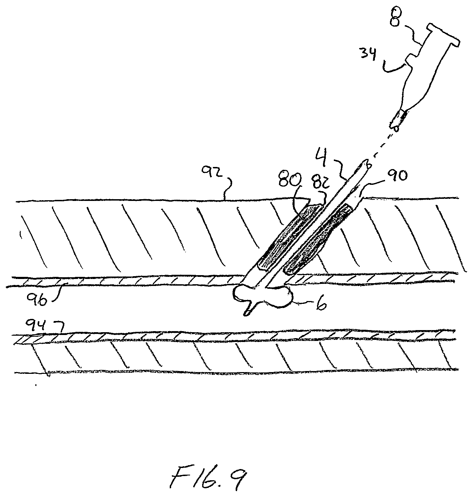

For example, FIG. 9 illustrates the apparatus 2 being used in connection with a plug or other carrier 80. The carrier 80 includes a lumen 82 therein sized to receive the elongate member 4 and expandable member 6 (in the contracted condition) therethrough. The carrier 80 may be formed from a lyophilized (i.e., freeze-dried) PEG polymer that contains hydrolytically degradable chemical groups, such as the plugs disclosed in co-pending application Serial Nos. 10/982,387 and 10/982,384, both filed November 5, 2004.

Turning to FIG. 8F, once sufficient sealing compound 146 is delivered, the sealing compound 146 may given sufficient time to at least partially (or fully) solidify, e.g., between about five and one hundred eighty (5-180) seconds. The balloon 6 may then be collapsed to the contracted condition and then withdrawn from the puncture 90.

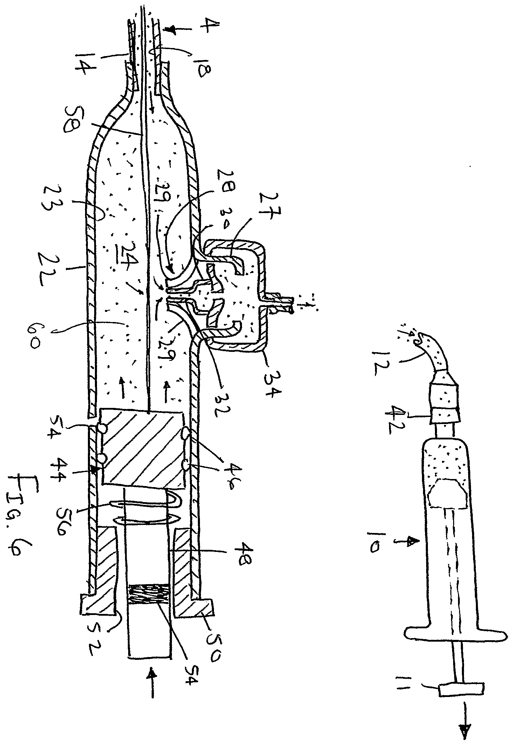

With reference to FIG. 6, a syringe 10 or other device (not shown) may be used to evacuate inflation media 60 from the balloon flow path to collapse the balloon 6. For example, the syringe 10 may be secured to the connector 42 on the end of the conduit 12, and the button 34 may be depressed to move the nipple 30 and fluid port 32 through the valve 28 into communication with the interior 24 of the housing 22. When the fluid port 32 is exposed to the interior 24 of the housing 22, a vacuum may be drawn on the syringe 10 by pulling plunger 11 to evacuate inflation media 60 from the interior 24, and therefore from the lumen 18 and interior 6c of the balloon 6 to deflate or otherwise collapse the balloon 6, as shown in FIG. 7. In addition, this vacuum may cause the piston 44 and pressure indicator 48 to retract into the hub subassembly 8 (i.e., move in the distal direction). In one embodiment, when the pressure indicator 48 is no longer visible (due to complete retraction inside the hub subassembly 8), the apparatus 2 may then be removed, knowing that the balloon 6 is sufficiently collapsed.

As the fluid is evacuated from the interior 24 of the housing 22, the bias of the piston 44 may cause the piston 44 to move distally, thereby lengthening the balloon 6 as it collapses. This may minimize the profile of the balloon 6 in the contracted condition, thereby facilitating removing the balloon 6 through the sealing compound 146 delivered into the puncture 90 without substantially disturbing the surrounding sealing compound 146.

To facilitate removing the balloon 6, a lubricious coating (not shown) may be provided on an exterior of the elongate member 4 and/or balloon 6, e.g., DOW 360 silicone fluid. Such a coating may prevent the sealing compound 146 from sticking to or otherwise pulling on the elongate member 4 and/or balloon 6 as the apparatus 2 is withdrawn.

Optionally, external pressure may be applied, e.g., by pressing manually against the skin 92 overlying the vessel 94, e.g., to at least partially suppress flow through the vessel 94. The balloon 6 (and the rest of the apparatus 2) may be removed, and the external pressure may be maintained for sufficient time to allow the sealing compound 146 to solidify further, e.g., between about ten and one hundred eighty (10-180) seconds. The sealing compound may expand, e.g., due to its elasticity and/or due to further solidification, thereby substantially sealing the relatively small tract remaining upon removing the apparatus 2.

Alternatively, a tensioner (not shown) may be used to maintain tension on the balloon 6 for a prolonged period of time with the balloon 6 providing temporary hemostasis to allow the hydrogel to cure fully in the puncture 90 before removing the apparatus 2.

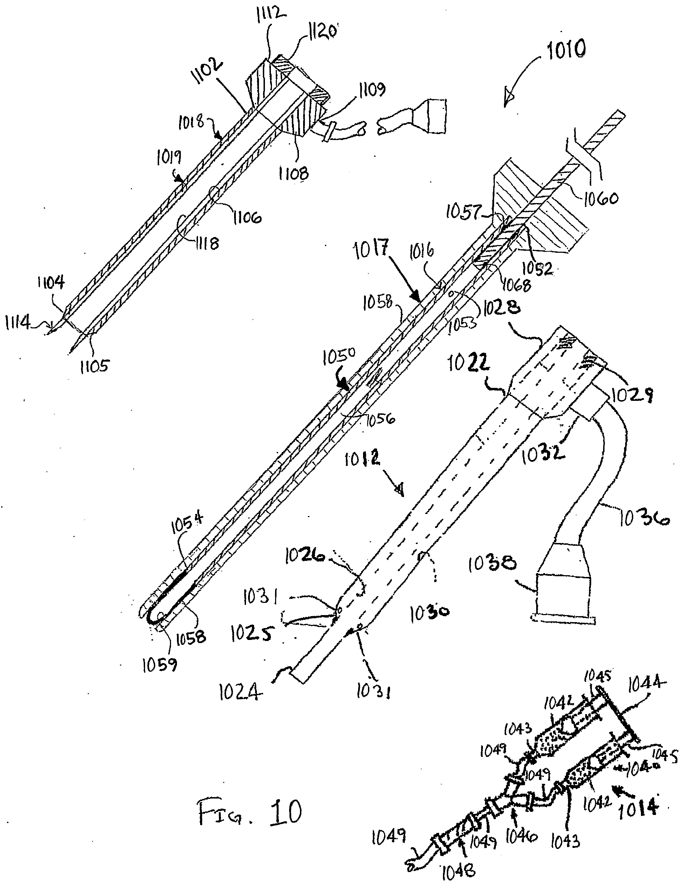

Turning to FIG. 10, another embodiment of a system 1010 is shown for accessing and/or delivering sealing compound into a puncture through tissue, e.g., a percutaneous puncture communicating from a patient's skin through intervening tissue to a blood vessel or other body lumen (not shown). Generally, the system 1010 includes a delivery or injection sheath 1012, a source of sealing compound 1014, and an occlusion member 1016. Optionally, the system 1010 may include other components, e.g., an introducer or procedure sheath 1018 (separate from the delivery sheath 1012), and one or more dilators (an exemplary dilator 1019 being shown). The system 1010 may also include one or more of a needle for creating the puncture, a guidewire, and/or one or more sections of tubing (not shown). In addition or alternatively, the system 1010 may include other components for creating the puncture, introducing the delivery sheath 1012 and/or guidewire into a body lumen, and/or accessing the vessel, e.g., for introducing instruments (not shown) into the vessel via the puncture.

Generally, the delivery sheath 1012 is an elongate tubular member, including a proximal end 1022, a distal end 1024, and a primary or guidewire lumen 1026 extending between the proximal and distal ends 1022, 1024. In addition, the delivery sheath 1012 may include one or more secondary or injection lumens 1030 that extend from the proximal end 1022 to one or more outlets (e.g., two outlets 1031, as shown) in the wall of the delivery sheath 1012.

As shown, a single secondary lumen 1030 is disposed concentrically around the primary lumen 1026. Alternatively, one or more secondary lumens (not shown) may be formed or otherwise provided in the wall of the delivery sheath 1012, e.g., in a side-by-side arrangement. The primary lumen 1026 may be of sufficient size to accommodate sliding a guidewire therethrough, e.g., between about 0.014 and 0.018 inch (0.35-0.45 mm) diameter, while the secondary lumen 1030 maybe of sufficient size to accommodate delivering sealing compound therethrough.

The secondary lumen 1030 extends from a housing 1028 on the proximal end 1022 of the delivery sheath 1012 to an intermediate portion 1025 between the proximal and distal ends 1022, 1024. As shown, the intermediate portion 1025 tapers where the secondary lumen 1030 terminates, with the delivery sheath 1012 having a smaller diameter from the intermediate portion 1025 to the distal end 1024 (e.g., since only the primary lumen 1026 extends along this portion of the delivery sheath 1012). The smaller diameter distal portion may have a desired length, e.g., at least about five millimeters (5 mm). The outlet(s) 1031 may be provided on the intermediate portion 1025, e.g., where the delivery sheath 1012 tapers, which may facilitate directing the sealing compound delivered through the secondary lumen 1030 radially outwardly away from the delivery sheath 1012.

The housing 1028 may be attached to or otherwise provided on the proximal end 1022 of the delivery sheath 1012. The housing 1028 may include one or more side ports (e.g., one side port 1032, as shown) that communicate with an interior of the housing 1028 and the secondary lumen 1030 of the delivery sheath 1012. The housing 1028 may include one or more seals 1029 to seal the interior of the housing 1028, e.g., such that sealing compound delivered from the side port 1032 may be directed through the secondary lumen 1030. Optionally, the housing 1028 may also include one or more seals (not shown), e.g., a hemostatic seal, for sealing the primary lumen 1026 while accommodating inserting a needle, guidewire, occlusion member, or other instrument (not shown) into the lumen 1026, e.g., preventing bodily fluids, such as blood, from escaping proximally through the delivery sheath 1012, as is known in the art.

A section of flexible tubing 1036 may be connected to or otherwise extend from the side port 1032 to a luer lock adapter 1038, a manual shut-off valve (not shown), and/or other connector (also not shown), e.g., to facilitate connecting tubing and the like (also not shown) to the side port 1032. For example, a source of sealing compound, such as the dual-syringe assembly 1040 described below, may be connected to the luer lock adapter 1038 before or during a procedure.

In alternative embodiments, the delivery sheath may be a tubular member including a single lumen (not shown), which may include a hub, side port, and/or other components, similar to the embodiment described above. Additional information on such delivery sheaths may be found in co-pending application Serial Nos. 10/454,362, filed June 4,2003 and 10/745,946, filed December 24, 2003.

Returning to FIG. 10, the occlusion member 1016 may include an elongate wire member or other tubular body 1050 carrying a balloon or other expandable member 1058. The wire member 1050 generally includes a proximal end 1052, a distal end 1054, and a lumen 1056 extending at least partially between the proximal and distal ends 1052, 1054. The wire member 1050 may have an outer diameter of about .008 to .038 inch, e.g., not more than about 0.40 inch. The wire member 1050 may be substantially flexible or semi-rigid, e.g., to allow the wire member 1050 to curve, bend, or otherwise adapt to anatomy through which it is advanced, yet have sufficient column strength to accommodate advancing the distal end 1054 by pushing on the proximal end 1052.

In one embodiment, the wire member 1050 may be formed from one or more wire coil(s) (not shown) wound into an elongate tubular shape, similar to a guidewire. The wire coil(s) may be formed from one or more substantially round, square, or flat wires, e.g., made from stainless steel, Nitinol, or other metal. Thus, the wire member 1050 may be substantially flexible yet may be pushed form the proximal end 1052 without substantial risk of kinking or buckling.

The wire member 1050 may include material applied to the wire coil(s), e.g., a polymer or other coating, to create a substantially nonporous wall such that fluid may be contained within the lumen 1056 without substantial leaking. For example, a liquid polymer or other material may be applied to the outer and/or inner surfaces of the wire coil(s), e.g., by dipping the entire wire coil(s), brushing, and/or spraying material onto the coiled wire(s), or by applying a coating to the wire(s) using such methods before the wire(s) are formed into the wire coil(s). If appropriate, the coating material may be cured or otherwise solidified using known procedures. Thus, coating material may have sufficient flexibility to allow the wire coil(s) to flex as the wire member 1050 bends while maintaining the coating integrity and nonporous wall.

In another embodiment, the wire member 1050 may be formed from a solid-walled tube, such as a section of thin-walled hypo-tube. Exemplary materials for the wire member 1050 include stainless steel, Nitinol, or other metal, polyimide or other plastic tubing, and/or composite materials.

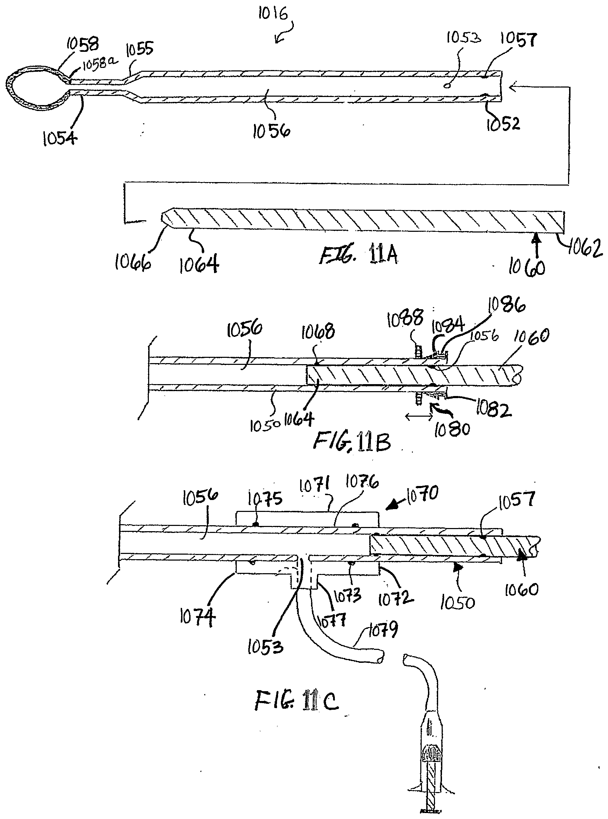

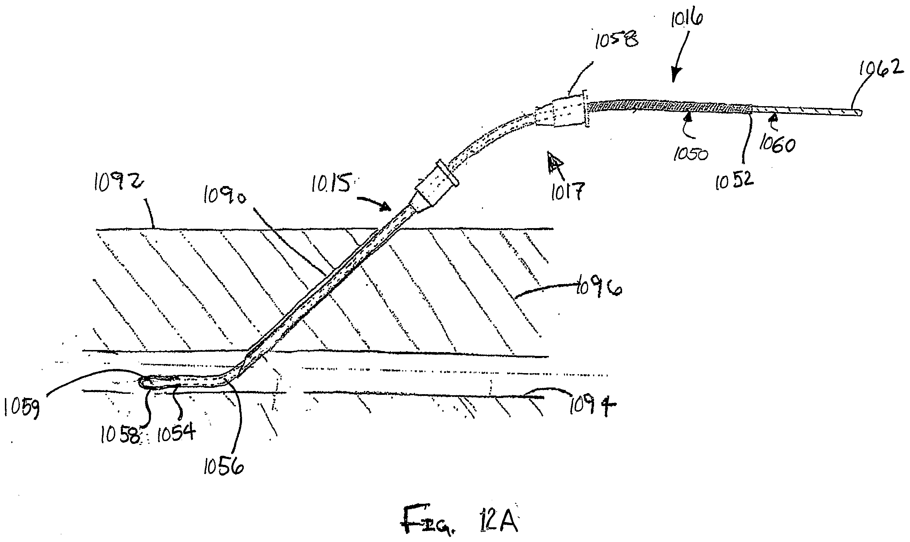

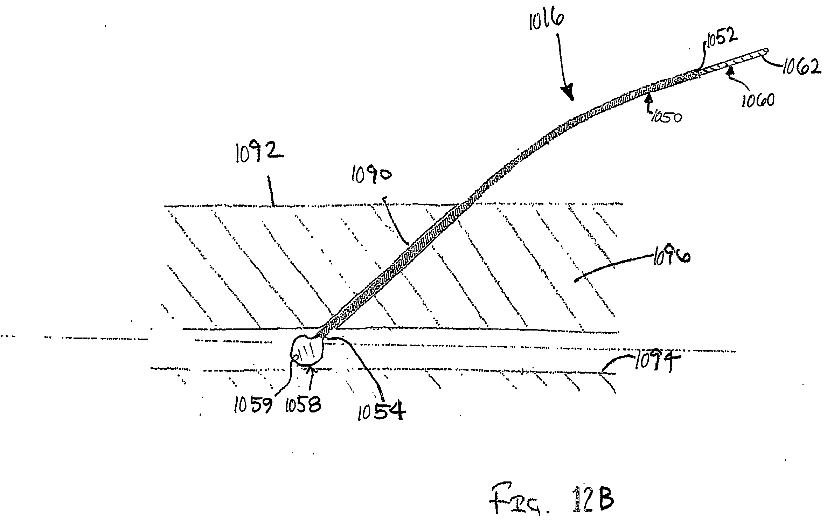

Returning to FIG. 10, with additional references to FIGS. 11A and 11B, the balloon 1058 may be expandable from a collapsed state (such as that shown in FIG. 10) to an enlarged state (such as that shown in FIG. 12A). For example, saline, air, or other fluid may be introduced into an interior 1059 of the balloon 1058 to expand the balloon 1058 from the collapsed state to the enlarged state.

In one embodiment, the balloon 1058 maybe formed from a flexible, substantially inelastic material, e.g., a nonelastomeric material, such as PET, nylon, polyethylene, polyurethane, PEBAX, and the like. Thus, the balloon 1058 may be substantially noncompliant or semi-compliant, thereby expanding to a predetermined size once a minimum pressure is introduced into the interior 1059 of the balloon 1058. Alternatively, the balloon 1058 may be formed from an elastic material, such that the size of the balloon 1058 in the expanded state is dependent upon the pressure or volume of fluid delivered into the balloon 1058. Additional information on balloons that may be used are disclosed in co-pending applications Serial No. 10/454,362 and Serial No. 10/806,927, filed March 22, 2004.

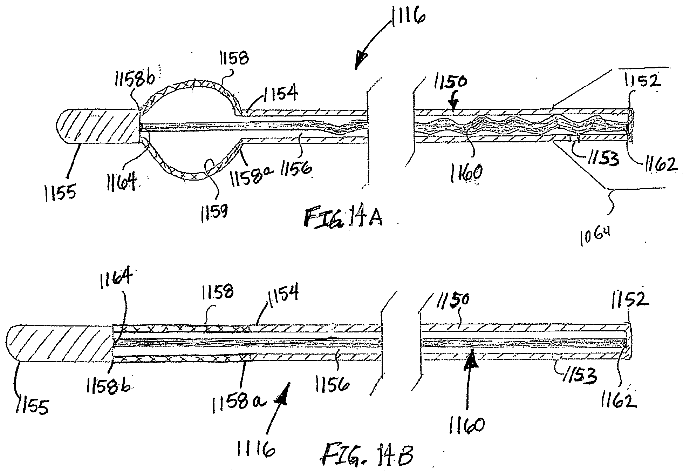

As shown in FIG. 11A, the balloon 1058 may be an enclosed expandable body with an open end 1058a. The open end 1058a maybe attached to the distal end 1054 of the wire member 1050 such that the balloon 1058 extends distally from the distal end 1054 of the wire member 1050. For example, the end 1058a may be bonded to the distal end 1054 and/or may be attached using a band (not shown) secured around the distal end 1054 and the open end 1058a of the balloon 1058. The interior 1059 of the balloon 1058 thereby communicates with the lumen 1056 of the wire member 1050 such that fluid from the lumen 1056 may be used to expand the balloon 1058, as explained further below. Alternatively, as seen in FIGS. 14A and 14B, an occlusion member 1116 may include a balloon 1158 disposed proximal to a distal tip 1155 of the occlusion member 1116, as described further below.

Returning to FIG. 10, with additional reference to FIGS. 11A-11C, the wire member 1050 may include one or more ports or other openings 1053 located in an intermediate region of the wire member 1050. The port(s) 1053 may simply be one or more holes formed in the side wall of the wire member 1050, e.g., by laser cutting, drilling, etching, and the like. The port(s) 1053 communicate with the lumen 1056, e.g., allowing a fluid source (not shown) to communicate with the lumen 1056 from the outside environment surrounding the wire member 1050.

The wire member 1050 also includes a rod, tube, or other piston 1060 slidable into and out of the lumen 1056 from the proximal end 1052 of the wire member 1050. The piston 1060 may be an elongate member including a proximal end 1062, and a distal end 1064 that is axially moveable within the lumen 1056 of the wire member 1050. The piston 1060 may be pushed or pulled, e.g., to either advance or retract the distal end 1064 within the lumen 1056 of the wire member 1050.

The piston 1060 may be formed from a substantially flexible or semi-rigid material, similar to the wire member 1050, e.g., having sufficient column strength to allow the distal end 1064 to be advanced into the wire member 1050 by pushing on the proximal end 1062. For example, the piston 1060 maybe an elongate wire coil, similar to the wire member 1050, or alternatively a solid wire or other filament. In one embodiment, the piston 1060 may have a substantially uniform profile along its entire length, e.g., having a diameter between about .005 to .035 inch, such that the distal end 1064 may be slidably received in the lumen 1054 of the wire member 1050.

Optionally, a lubricous, low-friction, or other coating (not shown) may be applied to an exterior surface of the piston 1060, e.g., on at least the distal end 1064. In addition or alternatively, a similar or different coating (also not shown) may be applied to at least a portion of an interior surface of the wire member 1050. The coating may reduce or increase the force needed to cause axial movement of the piston 1060 within the lumen 1056 of the wire member 1050, depending upon the resistance desired.

A fluid-tight seal may also be provided between the piston 1060 and the wire member 1050, e.g., to substantially seal the lumen 1056 of the wire member 1050 such that fluid within the lumen 1056 may not leak between the wire member 1050 and the piston 1060, e.g., proximally out the proximal end 1052 of the wire member 1050. For example, as shown in FIGS. 10 and 11A-11D, a seal 1068 may be provided on the distal end 1064 of the piston 1060, e.g., attached to the distal end 1064 and/or disposed on an exterior of the piston 1060 at or adjacent the distal end 1064. In addition or alternatively, a seal 1057 maybe provided on an interior surface of the wire member 1050. The seals 1057 and/or 1068 may provide a fluid-tight seal inside the wire member 1050 to retain fluid within the lumen 1056 and balloon 1058 once the distal end 1064 of the piston 1060 has moved distally past the port(s) 1053, as described further below.

Optionally, as seen in FIG. 10, the occlusion member 1016 may also include a retaining sheath or other constraint 1017 slidable over the wire member 1050, e.g., for maintaining the balloon 1058 in its collapsed condition and/or for facilitating advancing the occlusion member 1016 into a puncture through tissue. For example, the retaining sheath 1017 may be an elongate tubular member including proximal and distal ends, and a lumen extending therebetween. A hub may be located on the proximal end, e.g., to facilitate manipulating the retaining sheath 1017. The retaining sheath 1017 may have a diameter or other size to allow the distal end to be inserted into and/or through the primary lumen 1026 of the delivery sheath 1012. The hub of the retaining sheath 1017 maybe larger than the size of the primary lumen 1026, e.g., to provide a stop limiting distal advancement of the retaining sheath 1017 into the delivery sheath 1012. The retaining sheath 1058 maybe sufficiently flexible to conform to the surrounding anatomy, e.g., when the retaining sheath 1058 is inserted into or removed from a puncture, e.g., along with other components.

Referring to FIG. 11A, in one embodiment, the wire member 1050 may include a tapered region 1055 located proximal to the balloon 1058. Correspondingly, the distal end 1064 of the piston 1060 may include a shoulder or tapered distal tip 1066 angled to conform to the tapered region 1055 of the wire member 1050. The tapered region 1055 may act as an abutment to limit advancement of the piston 1060 within the wire member 1050. In addition, the shoulder 1066 may engage the tapered region 1055, e.g., by friction or interference fit, to restrict proximal movement of the piston 1060 relative to the wire member 1050, as explained further below.

Further, this configuration may provide tactile feedback to the user, e.g., that the balloon 1058 has been fully expanded. For example, the tapered region 1055 may be provided a predetermined distance from the port(s) 1053, thereby defining a volume of fluid within the lumen 1056 that may be directed into the balloon 1058 when the distal end 1064 of the piston 1060 is advanced from its loaded position (described below) into engagement with the tapered region 1055. The volume that is displaced may be determined to be the desired volume for sufficiently inflating and expanding the balloon 1058.

In alternative embodiments, the piston 1060 and/or wire member 1050 may include other lock elements (not shown) for securing the piston 1060 relative to the wire member 1050. For example, the piston 1060 may include one or more ramps, tabs, or other detents (not shown), e.g., on the distal end 1064, and the wire member 1050 may include one or more mating ramps, tabs, or other detents (also not shown), e.g., within the lumen 1056. When the piston 1060 is advanced a predetermined distance into the wire member 1050, e.g., until the distal end 1064 of the piston 1060 is disposed adjacent to the distal end 1054 of the wire member 1050 but proximal to the balloon 1058, the detents may interlock, preventing the piston 1060 from being withdrawn proximally.

For example, the lock element(s) may act as a ratchet, e.g., allowing the piston 1060 to be advanced distally but not retraced proximally until a final distal position is attained. Such lock element(s) may also provide tactile and/or audio feedback to the user, allowing the user to determine when the piston 1060 has reached a desired position, e.g., corresponding to complete expansion of the balloon 1058, as described further below. Optionally, the lock element(s) may be overcome by pulling on the piston 1060, e.g., using a predetermined force sufficient to disconnect or even break the lock securing the piston 1060. Once the lock is broken, the lock elements may prevent the piston 1060 from being reinserted into the wire member 1050, e.g., to prevent reuse of the occlusion member 1016.

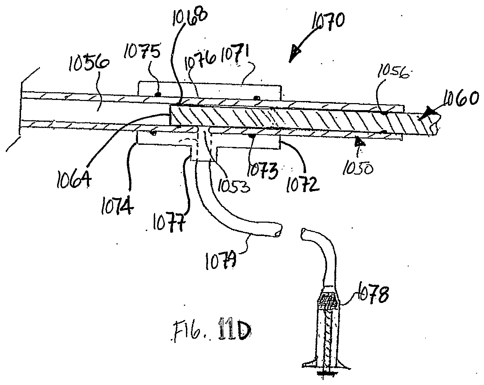

Turning to FIGS. 11C and 11D, a fluid loading device or housing 1070 is shown for loading fluid into the lumen 1056 of the wire member 1050. In the embodiment shown, the fluid loading device 1070 includes a barrel or other housing 1071 including first and second ends 1072, 1074, and a lumen 1076 extending between the first and second ends 1072, 1074. The housing 1071 also includes a side port 1077 communicating with the lumen 1076. In addition, the fluid loading device 1070 includes a source of vacuum and/or a source of fluid, such as one or more syringes (one syringe 1078 shown) connectable to the side port 1077 by tubing 1079. The fluid within the syringe 1078 may be a substantially incompressible liquid, such as water, saline, and the like, or a gas, such as air, nitrogen, carbon dioxide, and the like.

The housing 1071 has a size such that the housing 1071 may be received around the occlusion member 1016, e.g., by inserting one end of the wire member 1050 into the lumen 1076 of the housing 1070. The housing 1071 may include one or more seals to provide a fluid-tight seal between the housing 1071 and the wire member 1050. As shown, the housing 1071 includes an annular seal 1073, 1075 adjacent each of the first and second ends 1072, 1074 of the housing 1071, i.e., extending from an internal surface of the housing 1071 against the external surface of the wire member 1050.

Turning to FIGS. 11A-11D, a method is shown for loading fluid into the occlusion member 1016 using the fluid loading device 1070, e.g., before or during a medical procedure. As shown in FIG. 11A, the wire member 1050 and piston 1060 may be provided separately initially, e.g., in a kit along with one or more other components of the system 1010, such as the fluid loading device 1070 and/or the other components described herein. Alternatively, the occlusion member 1016 may be provided at least partially assembled, e.g., using one of the procedures described below, which maybe performed at a manufacturing location or at one or more other locations between the original manufacturing facility and the site of a procedure where the occlusion member 1016 may be used.

Turning to FIG. 11B, the occlusion member 1016 may be assembled by inserting the distal end 1064 of the piston 1060 into the proximal end 1052 of the wire member 1050. Consequently, the piston 1050 maybe directed into the lumen 1056, e.g., until the distal end 1064 of the piston 1060 is located proximal to the port(s) 1053 in the wire member 1050.

The housing 1071 of the fluid loading device 1070 may then be directed over the wire member 1050. The seals 1073, 1075 of the housing 1071 may slide along the exterior surface of the wire member 1050 as the housing 1071 is directed along the wire member 1050. When the housing 1071 overlies the port(s) 1053, the seals 1073, 1075 may straddle the port(s) 1053 such that the lumen 1076 of the housing 1071, and consequently the side port 1077, communicate with the lumen 1056 of the wire member 1050 via the port(s) 1053. Alternatively, the piston 1060 may be advanced into the wire member 1050 after the housing 1071 is placed around the wire member 1050 over the port(s) 1053, e.g., until the distal end 1064 is disposed proximal to the port(s) 1053.

With the housing 1071 and piston 1060 loaded on and in the wire member 1050, the lumen 1056 of the wire member 1050 may contain air or other gases. These gases may be evacuated from the lumen 1056 by connecting a source of vacuum to the side port 1077 of the housing 1071. For example, a syringe 1078 maybe connected, e.g., by tubing 1079 to the side port 1077. The syringe 1078 may then be drawn to substantially aspirate the air from within the lumen 1056 of the wire member 1050. Alternatively, a vacuum line and the like (not shown) may be used to evacuate the air out of the lumen 1056 and balloon 1058. Once the air is evacuated from the lumen 1056, a valve (not shown) on the tubing 1079 or a connector on the side port 1077 (not shown) may be closed to maintain the vacuum within the lumen 1056.

A source of fluid, e.g., another syringe filled with fluid (represented by syringe 1078, although a different syringe may be used), may be coupled to the side port 1077, e.g., via tubing 1079 for delivering fluid into the lumen 1056 of the wire member 1050. The syringe 1078 may be depressed to deliver sufficient fluid into the lumen 1056 via the port(s) 1053 to substantially fill the lumen 1056. The fluid is preferably delivered into the lumen 1056 in such a manner so as to not cause deployment or expansion of the balloon 1058, i.e., such that the balloon 1058 remains substantially collapsed.

Once the lumen 1056 has been sufficiently filled with fluid, the piston 1060 may be advanced distally until the distal end 1064 is disposed distal to the port(s) 1053. Because of the seal 1068 on the piston 1060, the lumen 1056 and interior 1059 of the balloon 1058 may become substantially isolated from the port(s) 1053 and therefore from the surroundings around the wire member 1050. The housing 1071 may then be removed from around the wire member 1050, e.g., by sliding the housing 1071 down and off the end of the wire member 1050, without fluid leaking.

The piston 1060 may only be advanced a relatively short distance, e.g., such that the seal 1068 is located just beyond the port(s) 1053. This may minimize any expansion of the balloon 1058, which may occur as the piston 1060 displaces fluid within the lumen 1056 into the interior 1059 of the balloon 1058. In addition or alternatively, the volume of fluid delivered into the lumen 1056 using the fluid loading device 1070 may be reduced slightly, i.e., to maintain a slight vacuum within the lumen 1056. When the distal end 1064 of the piston 1060 is advanced past the side port(s) 1053, the volume displaced may correspond to the residual vacuum. This may reduce the risk of the balloon 1058 expanding undesirably when the piston 1060 is advanced to isolate the lumen 1056.

Optionally, as shown in FIG. 11B, the occlusion member 1016 may include a locking device 1080, which may be used to selectively restrain the piston 1060 from moving axially relative to the wire member 1050. As shown, the locking device 1080 may include an annular member 1082 attached or otherwise fixed to the proximal end 1052 of the wire member 1050. The annular member 1082 may include a ramped distal surface 1084 leading to a recessed abutment 1086. The locking device 1080 may also a locking ring 1088 disposed distal to the annular member 1082 and slidable along an exterior surface of the wire member 1014. For example, the locking ring 1088 maybe directed axially, e.g., proximally, until it slidably engages the ramped distal surface 1084 and enters the recessed abutment 1086.

The locking ring 1088 may compress the annular member 1082 inwardly as it slides along the ramped distal surface 1084, thereby compressing the proximal end 1052 of the wire member 1050 inwardly against the piston 1055, e.g., to crimp the wire member 1050 against the piston 1060 or otherwise frictionally engage the wire member 1050 and piston 1060 together. Thus, substantial axial movement of the piston 1060, distally or proximally, relative to the wire member 1050 may be prevented using the locking device 1080.

To allow movement of the piston 1060, the locking ring 1088 may be disengaged from the recessed abutment 1086 by pushing the locking ring 1088 distally out of the recessed abutment 1084 and down the ramped distal surface 1086. Once the locking ring 1088 is disengaged, the piston 1060 may be free to move axially within the wire member 1050.

Alternatively, the locking device may include other locking mechanisms, such as one or more clips, retainers, and the like that may be activated to prevent substantial axial movement of the piston 1060 relative to the wire member 1050 while the locking device is engaged.

Returning to FIG. 10, the system 1010 may also include a source of sealing compound 1014, such as a dual syringe assembly 1040 or other delivery device (not shown), e.g., that includes two components of a sealing compound. As shown, the syringe assembly 1040 includes a pair of syringe barrels 1042, including outlets 1043 and a plunger assembly 1044 slidable into the barrels 1042 to cause the components therein to be delivered through the outlets 1043. In the embodiment shown, the plunger assembly 1044 includes a pair of plungers 1045 coupled to one another that are received in respective barrels 1042. In this manner, both plungers 1045 may be manually depressed substantially simultaneously to deliver the components together from the syringe barrels 1042. Alternatively, a system for automatically advancing the plungers 1045 and/or otherwise delivering the components in the barrels 1042 may be used, such as those disclosed in co-pending application Serial No. 10/806,934, filed March 22, 2004.

Optionally, the delivery device 1014 may include a "Y" fitting 1046, a static mixer 1048, and/or tubing 1049, e.g., for connecting the "Y" fitting 1048 to outlets 1043 of the barrels 1042, the mixer 1048 to the "Y" fitting 1046 and/or to the side port 1032 of the delivery sheath 1012, such that the sealing components ejected out of the barrels 1042 may mix before being directed into the side port 1032 of the delivery sheath 1012. The outlets 1043, "Y" fitting 1046, mixer 1048, and/or tubing 1049 may include cooperating connectors, e.g., luer lock connectors and the like (not shown), for connecting them together.

Respective sealing components may be provided in each syringe barrel 1042 of the syringe assembly 1040 that, when mixed together, are activated to form a hydrogel or other sealing compound. Additional information on such hydrogels and systems for delivering them are disclosed in U.S. Patent Nos. 6,152,943, 6,165,201, 6,179,862, 6,514,534, and 6,379,373, and in co-pending published applications US 2002/0106409 published August 8, 2002, US 2003/0012734, published January 16, 2003, US 2002/0114775 published August 22, 2002, and US 2004/0249342 published December 9, 2004.

With continued reference to FIG. 10, the system 1010 may also include an introducer sheath 1018. As shown, the introducer sheath 1018 is an elongate tubular member including a proximal end 1102, a distal end 1104, and a lumen 1106 extending between the proximal and distal ends 1102, 1104. The introducer sheath 1018 may terminate in a tapered distal tip 1105 for facilitating advancing the introducer sheath 1018 substantially atraumatically through tissue into a puncture. Exemplary materials for the introducer sheath 1018 may include one or more plastics, such as FEP, polyvinyl chloride (PVC), polyamide, PEEK, nylon, PET, PEBAX, and polyethylene, metals, such as stainless steel, and nickel titanium, and/or composite materials. The introducer sheath 1018 may be substantially rigid, semi-rigid, or substantially flexible, e.g., to facilitate insertion through a puncture into a blood vessel or other body lumen. The introducer sheath 1018 may have an outer diameter between about .080 to .140 inch and/or a wall thickness between about .002 to 0.10 inch.

A housing 1108 may be attached to or otherwise provided on the proximal end of the introducer sheath 1018. The housing may include a side port 1109 that communicates with an interior of the housing 1108 and the lumen 1106 of the introducer sheath 1018. A section of flexible tubing may be connected to or otherwise extend from the side port 1109, terminating in a manual shut-off valve and/or a luer lock or other connector (not shown), e.g., to facilitate connecting tubing and the like (not shown) to the side port 1109. The housing 1108 may also include one or more seals (not shown), e.g., a hemostatic seal, for substantially sealing the lumen of the delivery sheath 1018, yet accommodating inserting one or more instruments (not shown) into the lumen.

Optionally, a dilator 1019 may also be provided, e.g., within the lumen 1086 of the introducer sheath 1018. The dilator 1019 may also include a proximal end 1112, a distal end 1114 sized for insertion through the lumen of the introducer sheath 1018, a lumen 1118 extending between the proximal end distal ends, and a hub or other handle 1120 on the proximal end 1112. The distal end 1114 may include a tapered or multiple ramped shape, similar to known dilators. The dilator 1019 may be formed from substantially rigid, semi-rigid, or substantially flexible materials, similar to the introducer sheath 1018.

The dilator 1019 maybe loaded into the introducer sheath 1018 during manufacturing or immediately before a procedure. In addition, the dilator 1019 may be loaded into the introducer sheath 1018 by inserting the distal end 1114 of the dilator 1019 into the hub 1108 and lumen 1106 of the introducer sheath 1018 until the hubs 1108, 1120 abut one another. Once inserted into the introducer sheath 1018, the distal end 1114 of the dilator 1019 may extend beyond the distal end 1104 of the introducer sheath 1018, e.g., to provide a gradually tapering transition for the assembly. Thus, before a procedure, the dilator 1019 and introducer sheath 1018 may be disposed concentrically around one another in an assembly, as shown in FIG. 10. Optionally, the dilator 1019 maybe eliminated, if desired.

In one embodiment, a flexible and/or thin-walled sleeve 1020, similar to those disclosed in U.S. Application Serial No. 11/112,970, filed April 22, 2005, may also be used in connection with the introducer sheath 1018.

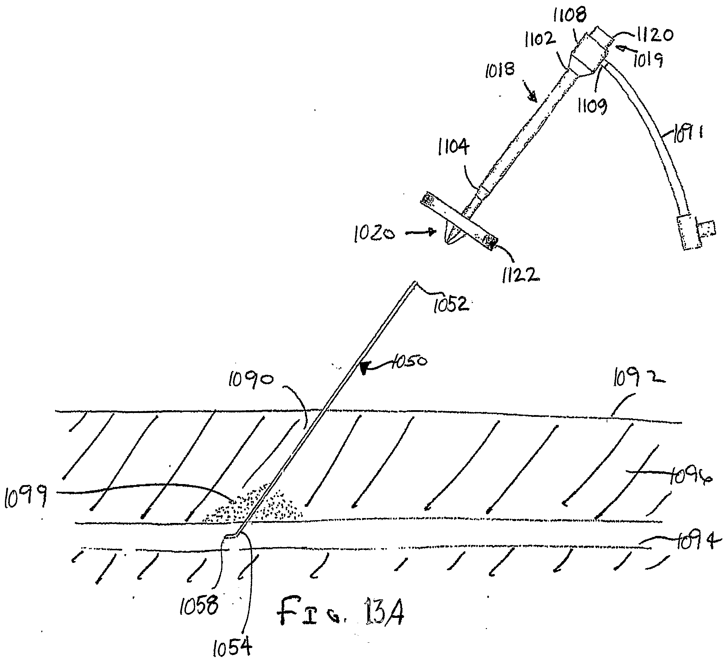

Turning to FIGS. 12A-12C and 13A-13C, a method is shown for delivering an introducer sheath (and/or sleeve), such as the introducer sheath 1018 described above, into a passage 1090 extending through tissue 1096. In the illustrated embodiment, the passage 1090 is a percutaneous puncture extending from a patient's skin 1092 to a blood vessel or other body lumen 1094. For example, the vessel 1094 may be a peripheral artery, e.g., a femoral artery, a carotid artery, and the like. It will be appreciated that systems and methods constructed and undertaken as described herein may be used to seal other passages through tissue within a patient's body.

Initially, as shown in FIGS. 12A-12C, the puncture 1090 may be created and sealing compound 1099 may be delivered into the puncture 1090. Turning to FIG. 11A, to create the puncture 1090, a hollow needle 1015 maybe inserted through the patient's skin 1092 and intervening tissue 1096 into the vessel 1094. The occlusion member 1016, e.g., with the wire member 1050 loaded with the piston 1060 sealing fluid therein, maybe inserted into the puncture 1090, e.g., through the needle 1015 until the distal tip 1066 is disposed within the vessel 1094. Optionally, as shown, the retaining sheath 1017 may cover the balloon 1058 on the wire member 1050 as the occlusion member 1016 is advanced through the needle 1015, thereby maintaining the balloon 1058 in the contracted or collapsed condition.

Turning to FIG. 11B, once the balloon 1058 is located within the vessel 1094, the needle may be removed, and the balloon 1058 may be expanded within the vessel 1094. For example, the retaining sheath 1017 may be retracted completely (or only partially) out of the puncture 1090 to expose the balloon 1058 within the vessel 1084.

The balloon 1058 may then be expanded within the vessel 1094. For example, as shown in FIG. 11B, the piston 1060 may be advanced distally relative to the wire member 1050. As the piston 1060 is advanced within the wire member 1050, fluid within the lumen 1056 may enter the interior 1059 of the balloon 1058, causing the balloon 1058 to expand. As described above, the wire member 1050 and/or piston 1060 may include one or more elements that tactile and/or audio feedback to provide an indication that the piston 1060 has been advanced to a position where the balloon 1058 is fully expanded.

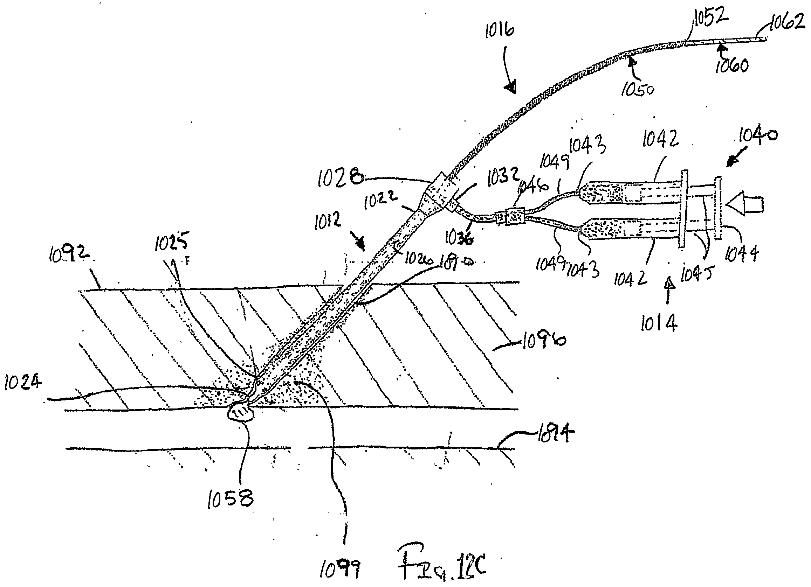

Turning to FIG. 12C, with the balloon 1058 fully expanded, a delivery sheath, such as the delivery sheath 1012 described above, may be advanced over the occlusion member 1016 into the puncture 1090, e.g., before or after the balloon 1058 is expanded. For example, the proximal end 1062 of the piston 1060 maybe backloaded into the distal end 1024 of the delivery sheath 1012, and then the delivery sheath 1012 maybe advanced over the piston 1060 and wire member 1050 until the distal end 1024 enters the vessel 1094. Because of the substantial uniformity in cross-section and size of the piston 1060 and wire member 1050, the delivery sheath 1012 may pass easily over the occlusion member 1016. Alternatively, the proximal end 1052 of the wire member 1050 may include a transition, e.g., a ramped proximal edge (not shown) to facilitate advancing the delivery sheath 1012 over the wire member 1050.

In one embodiment, the delivery sheath 1012 may be advanced until the distal end 1024 is disposed within the vessel 1094. The balloon 1058 may then be expanded (if not expanded before introducing the delivery sheath 1012), and the occlusion member 1016 may be partially retracted until the balloon 1058 contacts the distal end 1024 of the delivery sheath 1012 (providing a first tactile feedback). The occlusion member 1016 may be retracted by pulling on the proximal end 1052 of the wire member 1050 unless the piston 1060 is locked relative to the wire member 1050, whereupon the piston 1060 may be pulled. Otherwise, the piston 1060 may be retracted relative to the wire member 1050, which may prematurely deflate the balloon 1058 or even release the fluid within the lumen 1056 of the wire member 1050.

The occlusion member 1016 may then be pulled further until the balloon 1058 contacts the wall of the vessel 1094 (providing a second tactile feedback), thereby partially in retracting the delivery sheath 1012 back into the puncture 1090, e.g., until the distal end 1024 is disposed adjacent the vessel 1094.

Alternatively, the occlusion member 1016 maybe retracted until the occlusion element 1051 contacts the wall of the vessel 1094 before the delivery sheath 1012 is introduced. The delivery sheath 1012 may then be advanced into the puncture 1090 until the distal end 1024 contacts the expanded balloon 1058, thereby providing tactile feedback that the outlets 1025 of the delivery sheath 1012 are disposed within the puncture 1090 proximal to the vessel 1094.

With continued reference to FIG. 12C, a source of sealing compound 1014, e.g., the dual syringe assembly 1040 described above, may be prepared and connected to the side port 1032 of the delivery sheath 1012, e.g., via tubing 1049, either before or after the delivery sheath 1012 is advanced into the puncture 1090. The sealing compound 1099 may then be delivered through the secondary lumen 1030 and the outlets 1025 and into the puncture 1090. The sealing compound 1099 may flow radially outwardly to permeate at least partially into the tissue surrounding the puncture 1090.

Optionally, the delivery sheath 1012 may be retracted as the sealing compound 1099 is delivered, e.g., to fill the puncture 1090 along its length. Additional apparatus and methods for delivering the sealing compound 1099 into the puncture 1090 around the occlusion member 1016 are disclosed in co-pending application Serial Nos. 10/454,362 and 10/745,946, or in co-pending application Serial No. 10/975,205, filed October 27, 2004.

Once a desired amount of the sealing compound 1099 is delivered into the puncture 1090, the occlusion member 1016 maybe maintained such that the balloon 1058 continues to seal the puncture 1090 from the vessel 1094, e.g., for sufficient time for the sealing compound 1099 to at least partially or completely cure. Thereafter (or immediately after filling the puncture 1090), the delivery sheath 1012 maybe removed entirely from the puncture 1090.

The balloon 1058 may then be deflated and the occlusion member 1016 may be removed from the vessel 1094 and puncture 1090. In one embodiment, the balloon 1058 may be deflated by moving the piston 1060 proximally relative to the wire member 1050. This action may withdraw the fluid within the interior 1059 of the balloon 1058 back into the lumen 1056 of the wire member 1050, thereby substantially collapsing the balloon 1058. With the balloon 1058 collapsed, the occlusion member 1016 may simply be pulled proximally out through the puncture 1090.

If the piston 1060 is locked relative to the wire member 1050, the lock (not shown) may need to be disengaged. Alternatively, if the lock is not releasable, the piston 1060 may be pulled with sufficient force to break the lock. Optionally, the piston 1060 may be removed entirely from the wire member 1050, thereby releasing the fluid within the lumen 1056 of the wire member 1050 and exposing the lumen 1056 and interior 1059 of the balloon 1058 to the ambient pressure of the surroundings.

The wire member 1050 may be withdrawn before or after removing the delivery sheath 1012. If the delivery sheath 1012 remains within the puncture 1090 while the wire member 1050 is removed, the balloon 1058 maybe forced to collapse as it enters the delivery sheath 1012. If the piston has been removed or withdrawn proximally beyond the side port(s) 1053 in the wire member 1050, this action may force any residual fluid within the balloon 1058 out of the balloon 1058 and out the side port(s) 1053 and/or proximal end 1052 of the wire member 1050.

Optionally, turning now to FIGS. 13A-13C, an introducer sheath 1018 and/or flexible sleeve 1020, such as those described above and disclosed in the above-identified applications, maybe delivered into the puncture 1090 and/or through the sealing compound 1099. The sleeve 1020 may prevent sealing compound 1099 from separating and/or otherwise being released during introduction of the introducer sheath 1018, as explained further below. Alternatively, the sleeve 1020 may be omitted entirely.

As shown in FIG. 13A, the wire member 1050 (or the entire occlusion member 1016) may remain within the puncture 1090 and vessel 1094 after delivering the sealing compound 1099. Optionally, the balloon 1058 (not shown) may remain exposed and/or expanded, or the optional retaining sheath 1017 (also not shown) may be advanced over the wire member 1050 to cover and/or collapse the balloon 1058. Alternatively, the wire member 1050 may be removed from the puncture 1090 and a separate guidewire (not shown), e.g., without balloon 1058, may be advanced through the puncture 1090 into the vessel 1094 in place of the wire member 1050.

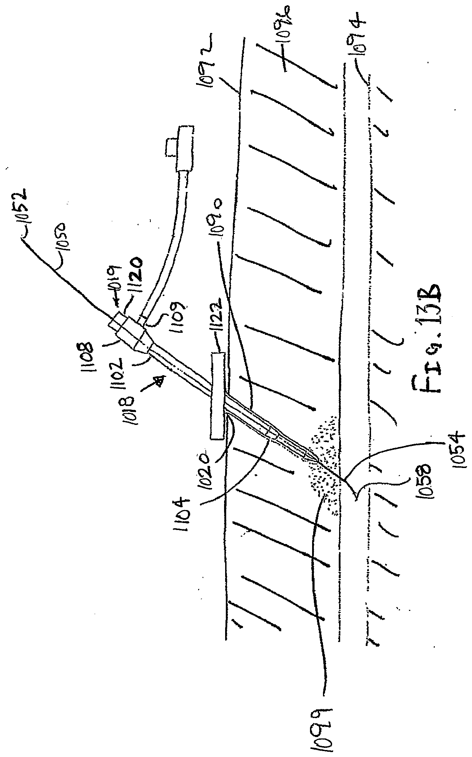

Turning to FIG. 13B, the introducer sheath 1018, dilator 1019, and sleeve 1020 may then be introduced into the puncture 90, e.g., over the wire member 1050 (or the entire occlusion member 1016). For example, the proximal end 1052 of the wire member 1050 and/or the proximal end 1062 of the piston 1060 may be backloaded into the introducer sheath 1018 before the introducer sheath 1018 is advanced into the puncture 1090. Optionally, hub 1122 of the sleeve 1020 may be placed against or immediately above the patient's skin 1092 overlying the puncture 1090.

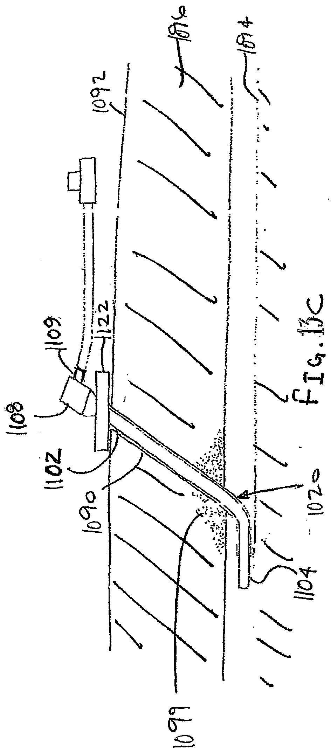

Turning to FIG. 13C, the introducer sheath 1018 (and any of the dilator 1019 and/or sleeve 1020 carried therein) may then be advanced into the puncture 1090, e.g., until the distal end 1104 of the introducer sheath 1018 is disposed within the vessel 1094. As the introducer sheath 1018 is advanced, the sleeve 1020 may unfurl from the introducer sheath 1018 and become exposed within the puncture 1090, e.g., everting and surrounding the exterior of the introducer sheath 1018. In the embodiment shown, the length of the sleeve 1020 is shorter than the introducer sheath 1018 such that the free end of the sleeve 1020 is disposed proximal to the distal end of the introducer sheath 1018 and may not extend into the vessel 1094.

The dilator 1019 and/or tubular member (not shown) may be withdrawn through the introducer sheath 1018 from the puncture 1090, e.g., together or successively, leaving the introducer sheath 1018 and sleeve 1020 within the puncture 1090. The wire member 1050 (or entire occlusion member 1016) may also be removed along with, before, or after the dilator 1019 and/or tubular member, e.g., after collapsing the balloon 1058 (not shown). Alternatively, if the balloon 1058 is still expanded, the wire member 1050 may be removed, causing the introducer sheath 1018 or dilator 1019 to collapse the balloon 1058 towards the collapsed state as it enters the lumen of the introducer sheath 1018.

Once the distal end of the introducer sheath 1018 is disposed within the vessel 1094, one or more instruments (not shown) may be advanced through the introducer sheath 1018 into the vessel 1094, e.g., to perform one or more diagnostic and/or interventional procedures within the patient's body, as is known to those skilled in the art. The sleeve 1020 generally does not interfere with the introduction of such instruments, since it is located only around the introducer sheath 1018.

Optionally, if the sleeve 1020 includes any weakened seams, the sleeve 1020 may be removed from around the introducer sheath 1018 to provide a conventional introducer sheath arrangement for the subsequent procedure. For example, the sleeve 1020 may separate into two or more pieces, e.g., along one or more predetermined seams (not shown). Thus, conventional procedures may be used without need for extra attention to the sleeve 1020.

Upon completing any such procedures, the instrument(s) may be removed from the vessel 1094 through the introducer sheath 1018. The introducer sheath 1018 and sleeve 1020 (if remaining around the introducer sheath 1018) may then be removed from the vessel 1094 and puncture 1090, e.g., simultaneously or successively. The sealing compound 1099 and/or tissue may recoil sufficiently to substantially fill the puncture 1090, thereby allowing and/or encouraging hemostasis to occur between the vessel 1094 and puncture 1090. Optionally, external pressure maybe applied to the patient's skin 1092 during removal of the introducer sheath 1018, e.g., to further enhance sealing of the puncture 1090 until hemostasis occurs.

Turning to FIGS. 14A and 14B, another embodiment of an occlusion member 1116 is shown that may be included in a system, e.g., instead of the occlusion member 1016 described above. The occlusion member 1016 generally includes an elongate wire member or other tubular body 1150 carrying a balloon or other expandable member 1158. Similar to the previous embodiment, the wire member 1150 includes a proximal end 1152, a distal end 1154, and a lumen 1156 extending at least partially between the proximal and distal ends 1152,1154. The wire member 1150 may have an outer diameter or other cross-section between about .008-.038 inch, e.g., not more than about 0.040 inch.

The wire member 1150 may be substantially flexible or semi-rigid, e.g., to allow the wire member 1150 to curve, bend, or otherwise adapt to anatomy through which it is advanced, yet have sufficient column strength to accommodate advancing the distal end 1154 by pushing on the proximal end 1152. The wire member 1150 may be formed from one or more wire coil(s) (not shown) wound into an elongate tubular shape, optionally, including applied to the wire coil(s) to create a substantially nonporous wall or may be formed from a solid-walled tube, similar to the previous embodiment.

The balloon 1158 may be expandable from a collapsed state (such as that shown in FIG. 14B) to an enlarged state (such as that shown in FIG. 14A), e.g., by introducing into an interior 1159 of the balloon 1158. The balloon 1158 may be formed from a flexible, substantially inelastic material, e.g., to provide a substantially noncompliant or semi-compliant balloon 1159 that expands to a predetermined size, or the balloon 1158 may be formed from an elastic material, such that the size of the balloon 1158 depends upon the pressure or volume of fluid delivered into the balloon 1158.

As shown, the balloon 1158 is disposed proximal to a distal tip 1155 of the occlusion member 1116, as described further below. For example, the balloon 1158 may include a first end 1158a attached to a distal end 1154 of a wire member 1150 and a second end 1158b attached to the distal tip 1155. The distal tip 1155 may be a rounded, tapered, and/or substantially blunt member providing a substantially atraumatic tip, e.g., including a "J" tip (not shown), if desired to facilitate advancement.

As shown, the proximal end 1152 of the wire member 1150 is substantially closed, e.g., by providing a plug, sealant, glue, or other seal material in the proximal end 1152. Alternatively, the proximal end 1152 may be capped using a cap or other element (not shown). Thus, the lumen 1156 may be substantially isolated from the region surrounding the wire member 1150.

To communicate with the lumen 1156, the wire member 1150 may include one or more ports or other openings 1153 located in an intermediate region of the wire member 1150, e.g., adjacent the proximal end 1152. The port(s) 1153 may communicate directly with the lumen 1156, e.g., allowing a fluid source (not shown) to communicate with the lumen 1156 from the outside environment surrounding the wire member 1150, as described further below.

The occlusion member 1116 may also include a spring or biasing mechanism, such as spring wire 1160 within the lumen 1156 or otherwise coupled to the wire member 1150. The spring wire 1160 may be formed from an elastic or superelastic material, e.g., stainless steel or Nitinol, that may be sufficiently flexible to bend within the wire member 1150, yet have sufficient column strength to bias the spring wire 1160 to extend axially. The spring wire 1160 may be a solid wire, e.g., having a round or flat cross-section. Optionally, one or more other biasing mechanisms (not shown) may be provided or the biasing mechanism may be omitted.

The spring wire 1160 may be an elongate member including a proximal end 162 that is fixed relative to the wire member 1150, and a distal end 164 that is fixed relative to the distal tip 1155. For example, the proximal end 1162 of the spring wire 1160 may be attached to the proximal end 1162 of the wire member 1150, e.g., using an adhesive, sonic or other welding, embedding the proximal end 1162 in the seal material, and the like. The distal end 1164 of the spring wire 1160 may be similarly attached to the distal tip 1155 or alternatively to the distal end 1158b of the balloon 1158.

The relative lengths and fixation points of the wire member 1150 and the spring wire 1160 may place the spring wire 1160 under slight tension inside the wire member 1050, thereby biasing the distal tip 1155 away from the distal end 1154 of the wire member 1150. This may place the balloon 1158 under tension, thereby minimizing the cross-section of the balloon 1158 in the collapsed state. This bias provided by the spring wire 1160 may facilitate collapsing the balloon 1158 after a procedure, e.g., to facilitate withdrawing the balloon 1158 from a puncture 1090 (not shown), as described further below.

Turning to FIG. 12A, when fluid is delivered into the interior 1159 of the balloon 1158, e.g., using the devices and/or methods described further below, the balloon 1158 may shorten as it expands. Consequently, this may place the spring wire 1160 under a compressive stress, foreshortening the distance between the proximal and distal ends 1162, 1164 of the spring wire 1160. To reduce the resulting stress, the spring wire 1160 may partially coil or otherwise contort inside the lumen 1156 of the wire member 1150. This may cause the wire member 1150 to curve or otherwise twist to reduce the stress imposed by the spring wire 1160 when the wire member 1150 is free from outside forces or constraints (such as tissue surrounding a puncture).

When fluid 1054 is evacuated from the interior of the balloon 1158, the balloon returns towards the collapsed state of FIG. 14B. This may release the compressive force on the spring wire 1160, whereupon the spring wire 1160 may resiliently extend, i.e., move the distal end 1164 distally away from the proximal end 1162. This may bias the balloon 1158 to extend distally, i.e., elongate, as it returns towards the collapsed state, thereby minimizing the profile of the balloon 1158.

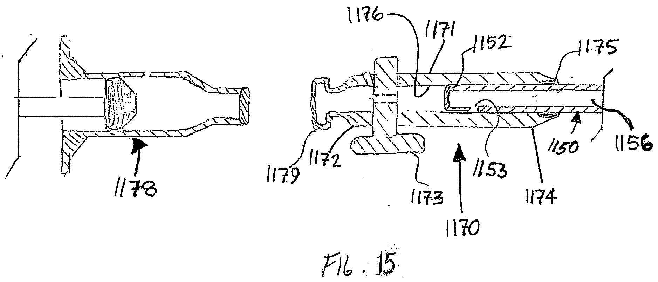

Turning to FIG. 15, an exemplary embodiment of a fluid loading device 1170 is shown for delivering fluid into and out of the lumen 1156 of the wire member 1150, e.g., via the one or more side port(s) 1153. The fluid may be used to selectively expand and/or collapse the balloon 1058, similar to the embodiments described above. Generally, the fluid loading device 1170 includes a housing 1171 receivable on the proximal end 1152 of the wire member 1150, and a connector 1179 for connecting to a source of vacuum or fluid, such as syringe 1178, to the housing 1171.

As shown, the housing 1171 includes a proximal end 1172 including the connector 1179 thereon, a distal end 1174, and a lumen or chamber 1176 extending at least partially between the proximal and distal ends 1172, 1174. The distal end 1174 is substantially open, having a size for receiving the proximal end 1152 of the wire member 1150 therein through the distal end 1174 of the housing 1171. The housing 1171 may also include a shut-off mechanism 1175 for selectively isolating or accessing the chamber 1176 from the proximal end 1172 of the housing 1171.

The housing 1171 has a sufficient length such that, when the housing 1171 is advanced onto the proximal end 1152 of the wire member 1150, the distal end 1174 of the housing 1171 extends distally beyond the side port(s) 1153 in the wire member 1150. Thus, the side port(s) 1153 in the wire member 1150 may communicate with the chamber 1176 around the proximal end 1152 of the wire member 1150. Optionally, the housing 1171 may include a seal 1175, such as a septum, o-ring, and the like, disposed within the distal end 1174 of the housing. The seal 1175 may be able to slide along the exterior of the wire member 1150, yet create a substantially fluid-tight seal between the exterior of the wire member 1150 and the distal end 1174 of the housing 1171.

The shut-off mechanism 1175 may include a manual stopcock that may be rotated to allow a passageway therein to communicate between the proximal end 1172 and the chamber 1176 or isolate the chamber 1176 from the proximal end 1172 depending on its orientation (shown as open in FIG. 15). Alternatively, a valve or other manual or actuated shut-off mechanism (not shown) may be provided instead of the stopcock. The connector 1179 may be a male or female Luer lock connector, or other connector for removably engaging with the syringe 1178. Alternatively, the connector 1179 and shut-off mechanism 1175 may be replaced by a penetrable seal (not shown), e.g., which may be penetrated by a needle (also not shown) on the syringe 1178.

Optionally, a locking mechanism (not shown) may be provided for securing the housing 1171 to the proximal end 1152 of the wire member 1150. For example, a clamp or other device may be provided that may be tightened around the distal end 1174 of the housing 1171. Alternatively, the housing 1171 may be sufficiently secured over the wire member 1150 by friction or an interference fit.

Still referring to FIG. 15, during use, the housing 1171 maybe advanced onto the wire member 1150, and a syringe 1178 may be connected to the connector 1179. With the shut-off mechanism 1173 open, the syringe 1178 maybe drawn to evacuate substantially the air out of the lumen 1156 of the wire member 1150 and/or out of the interior 1159 of the balloon 1158. Fluid, e.g., saline or water, may then be delivered into chamber 1176, and consequently into the lumen 1156 and the interior 1159 of the balloon 1158 to expand the balloon 1158. The shut-off mechanism 1173 may be closed, and the syringe 1178 may be disconnected, leaving the housing 1171 on the proximal end 1152 of the wire member 1150.

When it is desired to collapse the balloon 1158, the shut-off mechanism 1173 may be opened, thereby allowing the fluid within the lumen 1156 and the interior 1159 of the balloon 1158 to escape, e.g., to equalize the pressure within the balloon 1158 to the surrounding ambient pressure. Alternatively, a syringe 1178 or other source of vacuum may be connected to the connector 1179, and the fluid may be evacuated actively from within the balloon 1178 to collapse the balloon 1178.

During a procedure, such as the sealing procedure described above, the occlusion member 1116 may be advanced into a puncture (not shown), e.g., by introducing the distal end 1154 of the wire member 1150 into the puncture with the balloon 1158 collapsed until the balloon 1158 is disposed within a blood vessel (also not shown) accessed via the puncture. The occlusion member 1116 may be advanced through a needle (not shown) used to create the puncture or through a delivery sheath (also not shown) already placed in the puncture. If a delivery sheath is not already in place, the delivery sheath may be advanced over the occlusion member 1116, e.g., by backloading the proximal end 1152 of the wire member 1150 into the delivery sheath lumen.

The housing 1171 may then be attached to the proximal end 1152 of the wire member 1150, which should be extending from the proximal end of the delivery sheath. Alternatively, the housing 1171 may be attached to the proximal end 1152 of the wire member 1150 before the delivery sheath is introduced if the cross-section of the housing 1171 is small enough to pass through the delivery sheath lumen.

The syringe 1178 or other source of fluid may then be connected to the housing 1171, e.g., after opening the shut-off mechanism 1173, and fluid may be directed into the lumen 1156 of the wire member 1150 via the chamber 1176 of the housing 1171. Sufficient fluid may be introduced into the lumen 1156 of the wire member 1150 to substantially expand the balloon 1158 to the enlarged state.

The expanded balloon 1158 may then be used to seal the vessel from the puncture, e.g., by at least partially retracting the occlusion member 1116 until the balloon 1158 substantially engages the wall of the vessel. Sealing compound may then be delivered into the puncture via the delivery sheath, similar to the embodiments described above. Optionally, once sufficient sealing compound has been delivered into the puncture, the delivery sheath may be withdrawn from the puncture, and an introducer sheath (not shown) may be advanced over the occlusion member 1116 into the puncture.

The balloon 1158 may be collapsed and/or removed from the puncture, e.g., before or after placing the introducer sheath in the puncture. To collapse the balloon 1158, the shut-off mechanism 1173 may simply be opened, thereby allowing fluid to pass out of the interior 1159 of the balloon 1158, through the lumen 1156, and possibly out of the proximal end 1172 of the housing 1171. Fluid may be affirmatively evacuated from within the balloon 1158, or, with the shut-off mechanism 1173 open, the occlusion member 1116 may be withdraw, causing the balloon 1158 to collapse as it pulled into the lumen of the introducer or delivery sheath.

While the above-described occlusion members have been described in connection with pre-sealing applications, it should be understood that the occlusion members described herein may be used during other procedures. For example, in one embodiment, an occlusion member, such as those described above, may be inserted into a puncture through tissue after completing a procedure that involves accessing a blood vessel or other body lumen via the puncture. Exemplary apparatus and methods for accessing a blood vessel or other body lumen, and/or for sealing the puncture after completing such a procedure are described in co-pending application Serial Nos. 10/454,362, filed June 4, 2003, and 10/806,952, filed March 22, 2004.

While the invention is susceptible to various modifications, and alternative forms, specific examples thereof have been shown in the drawings and are herein described in detail. It should be understood, however, that the invention is not to be limited to the particular embodiments disclosed, but to the contrary, the invention is to cover all modifications, equivalents and alternatives falling within the scope of the appended claims.

## Claims

1. An apparatus (2) for providing hemostasis within a puncture extending through tissue, comprising:
an elongate member (4) comprising proximal and distal ends (14, 16) defining a longitudinal axis therebetween, and a lumen (18) extending between the proximal and distal ends (14, 16);
an expandable member (6) carried on the distal end (16) of the elongate member (4);
a housing (8) on the proximal end (14) of the elongate member (14), the housing (8) including an interior (24) communicating with the lumen (18) of the elongate member (4);
a pressure indicator (48) on the housing (8) for providing feedback indicating that sufficient fluid has been delivered into the interior to expand the expandable member (6) to a predetermined expanded condition; and
a piston (44) slidable within the housing interior (24) from a first position to a second position as fluid is delivered into the interior (24) of the housing (8), wherein the pressure indicator (48) is coupled to the piston (44),
the apparatus further **characterized by**:
the pressure indicator further comprising a shaft or member (48) that projects from the piston (44) outwardly from a proximal end (50) of the housing (8), the shaft or member (48) carrying a visual indicator (54) that provides a visual indication that a predetermined pressure and/or volume of fluid has been introduced into the housing (8) to expand the expandable member (6) to the predetermined expanded condition; and
a pressure relief mechanism (54) allowing fluid to escape from the interior of the housing when a predetermined pressure is exceeded, the pressure relief mechanism comprising an evacuation port (54) that communicates with the housing interior (24) when the piston (44) is at or proximal of a predetermined position to thereby prevent overexpansion of the expandable member (6).

2. The apparatus (2) of claim 1, further comprising a valve assembly (26) comprising a one-way valve (28) allowing access into the housing interior (24) upon application of a pressure differential across the valve (28), the valve assembly further comprising a movable plunger (30) for overriding and opening the valve (28) to allow access into the housing interior (24).

3. The apparatus (2) of claim 1 or 2, wherein the visual indicator (54) includes a series of graduations or other indicia that may be used to ascertain the degree of expansion of the expandable member (6).

4. The apparatus (2) of any preceding claim, wherein the visual indicator (54) is exposed from the housing (8) when the expandable member (6) is expanded to the predetermined expanded condition.

5. The apparatus (2) of any preceding claim, wherein the piston (44) is biased to the first position.

6. The apparatus (2) of any preceding claim, further comprising a connecting member (58) coupled to the piston (44) and extending to a location distal to the expandable member (6), such that a distal end (6a) of the expandable member (6) is directed proximally as the piston (44) moves from the first position to the second position.

7. The apparatus (2) of claim 6, wherein the piston (44) is biased to the first position, thereby biasing the connecting member (58) distally to subject the expandable member (6) to axial tension and thereby minimize a profile of the expandable member (6) in a contracted condition.

8. The apparatus (2) of claim 2, further comprising an actuator (34) coupled to the plunger (30) for moving the plunger (30) between a first position, which does not override the valve (28), and a second position, which overrides and opens the valve (28), and in which a fluid port may be extended through the valve (28) and in communication with the housing interior (24).

9. The apparatus (2) of claim 8, the actuator (34) being movable inwardly relative to the housing (8) to move the plunger (30) from the first position to the second position, the actuator (34) being biased outwardly such that the plunger (30) returns to the first position when the actuator (34) is released relative to the housing (8).

10. The apparatus (2) of claim 8 or 9, wherein the plunger (30) is alternately securable in the first and second positions upon activating and deactivating the actuator (34).

11. The apparatus of claim 2, 8, 9 or 10, wherein the valve (28) is a duckbill valve.

12. The apparatus (2) of any preceding claim, further comprising an elongate sheath (62) comprising proximal and distal ends (64,65), and a lumen (66) extending therebetween, the lumen (66) sized for receiving the elongate member (4) therethrough when the expandable member (6) is in a contracted condition.

13. The apparatus of claim 12, further comprising a sealing compound (146) deliverable through the elongate sheath (62) around the elongate member (4).

## Patentansprüche

1. Vorrichtung (2) zum Gewährleisten einer Blutstillung innerhalb einer Punktion, die sich durch Gewebe erstreckt, wobei die Vorrichtung Folgendes umfasst:
ein längliches Element (4), das ein proximales und ein distales Ende (14, 16) umfasst, die eine Längsachse zwischen denselben definieren, und ein Lumen (18), das sich zwischen dem proximalen und dem distalen Ende (14, 16) erstreckt,
ein ausdehnbares Element (6), das an dem distalen Ende (16) des länglichen Elements (4) getragen wird,
ein Gehäuse (8) an dem proximalen Ende (14) des länglichen Elements (4), wobei das Gehäuse (8) ein Inneres (24) einschließt, das mit dem Lumen (18) des länglichen Elements (4) in Verbindung steht,
einen Druckanzeiger (48) an dem Gehäuse (8) zum Bereitstellen einer Rückmeldung, die anzeigt, dass ausreichend Fluid in das Innere zugeführt worden ist, um das ausdehnbare Element (6) bis zu einem vorbestimmten ausgedehnten Zustand auszudehnen, und
einen Kolben (44), der innerhalb des Gehäuseinneren (24) von einer ersten Stellung zu einer zweiten Stellung verschiebbar ist, wenn Fluid in das Innere (24) des Gehäuses (8) zugeführt wird, wobei der Druckanzeiger (48) an den Kolben (44) gekoppelt ist,
wobei die Vorrichtung ferner durch Folgendes gekennzeichnet ist:
dass der Druckanzeiger ferner einen Schaft oder ein Element (48) umfasst, das von dem Kolben (44) nach außen von einem proximalen Ende (50) des Gehäuses (8) vorspringt, wobei der Schaft oder das Element (48) einen visuellen Anzeiger (54) trägt, der eine visuelle Anzeige bereitstellt, dass ein vorbestimmter Druck und/oder vorbestimmtes Volumen an Fluid in das Gehäuse (8) eingeleitet worden ist, um das ausdehnbare Element (6) bis zu dem vorbestimmten ausgedehnten Zustand auszudehnen, und
einen Druckentlastungsmechanismus (54), der es ermöglicht, dass Fluid aus dem Inneren des Gehäuses entweicht, wenn ein vorbestimmter Druck überschritten wird, wobei der Druckentlastungsmechanismus eine Entleerungsöffnung (54) umfasst, die mit dem Gehäuseinneren (24) in Verbindung steht, wenn sich der Kolben (44) an oder proximal von einer vorbestimmten Stellung befindet, um dadurch eine Überdehnung des ausdehnbaren Elements (6) zu verhindern.

2. Vorrichtung (2) nach Anspruch 1, die ferner eine Ventilbaugruppe (26) umfasst, die ein Einwegventil (28) umfasst, das auf das Anwenden eines Druckgefälles über das Ventil (28) hin einen Zugang in das Gehäuseinnere (24) ermöglicht, wobei die Ventilbaugruppe ferner einen beweglichen Druckkolben (30) zum Übersteuern und Öffnen des Ventils (28), um einen Zugang zu dem Gehäuseinneren (24) zu ermöglichen, umfasst.

3. Vorrichtung (2) nach Anspruch 1 oder 2, wobei der visuelle Anzeiger (54) eine Reihe von Gradeinteilungen oder anderen Kennzeichen einschließt, die verwendet werden können, um den Grad der Ausdehnung des ausdehnbaren Elements (6) zu ermitteln.

4. Vorrichtung (2) nach einem der vorhergehenden Ansprüche, wobei der visuelle Anzeiger (54) aus dem Gehäuse (8) freigelegt wird, wenn das ausdehnbare Element (6) bis zu dem vorbestimmten ausgedehnten Zustand ausgedehnt ist.

5. Vorrichtung (2) nach einem der vorhergehenden Ansprüche, wobei der Kolben (44) zu der ersten Stellung vorgespannt wird.

6. Vorrichtung (2) nach einem der vorhergehenden Ansprüche, die ferner ein Verbindungselement (58) umfasst, das an den Kolben (44) gekoppelt ist und sich bis zu einer Position, distal zu dem ausdehnbaren Element (6), erstreckt, so dass ein distales Ende (6a) des ausdehnbaren Elements (6) in Proximalrichtung gerichtet wird, wenn sich der Kolben (44) von der ersten Stellung zu der zweiten Stellung bewegt.

7. Vorrichtung (2) nach Anspruch 6, wobei der Kolben (44) zu der ersten Stellung vorgespannt wird, wodurch das Verbindungselement (58) in Distalrichtung vorgespannt wird, um das ausdehnbare Element (6) einer axialen Spannung auszusetzen und dadurch ein Profil des ausdehnbaren Elements (6) in einem zusammengezogenen Zustand zu minimieren.

8. Vorrichtung (2) nach Anspruch 2, die ferner einen Stellantrieb (34) umfasst, der an den Druckkolben (30) gekoppelt ist, um den Druckkolben (30) zwischen einer ersten Stellung, die das Ventil (28) nicht übersteuert, und einer zweiten Stellung, die das Ventil (28) übersteuert und öffnet und in der sich eine Fluidöffnung durch das Ventil (28) und in Verbindung mit dem Gehäuseinneren (24) erstrecken kann, zu bewegen.

9. Vorrichtung (2) nach Anspruch 8, wobei der Stellantrieb (34) nach innen im Verhältnis zu dem Gehäuse (8) beweglich ist, um den Druckkolben (30) von der ersten Stellung zu der zweiten Stellung zu bewegen, wobei der Stellantrieb (34) derart nach außen vorgespannt wird, dass der Druckkolben (30) zu der ersten Stellung zurückkehrt, wenn der Stellantrieb (34) im Verhältnis zu dem Gehäuse (8) freigegeben wird.

10. Vorrichtung (2) nach Anspruch 8 oder 9, wobei der Druckkolben (30) auf ein Aktivieren und Deaktivieren des Stellantriebs (34) hin abwechselnd in der ersten und der zweiten Stellung zu befestigen ist.

11. Vorrichtung (2) nach Anspruch 2, 8, 9 oder 10, wobei das Ventil (28) ein Entenschnabelventil ist.

12. Vorrichtung (2) nach einem der vorhergehenden Ansprüche, die ferner eine längliche Hülse (62) umfasst, die ein proximales und ein distales Ende (64, 65) und ein Lumen (66), das sich zwischen denselben erstreckt, umfasst, wobei das Lumen (66) zum Aufnehmen des länglichen Elements (4) durch dasselbe, wenn sich das ausdehnbare Element (6) in einem zusammengezogenen Zustand befindet, bemessen ist.

13. Vorrichtung (2) nach Anspruch 12, die ferner eine Dichtungsmasse (146) umfasst, die durch die längliche Hülse (62) um das längliche Element (4) zugeführt werden kann.

## Revendications

1. Appareil (2) destiné à entraîner une hémostase dans une ponction s'étendant à travers des tissus, comprenant :
un élément allongé (4), comprenant des extrémités proximale et distale (14, 16) définissant un axe longitudinal entre elles, et une lumière (18) s'étendant entre les extrémités proximale et distale (14, 16) ;
un élément dilatable (6) supporté sur l'extrémité distale (16) de l'élément allongé (4) ;
un logement (8) sur l'extrémité proximale (14) de l'élément allongé (14), le logement (8) englobant un intérieur (24) communiquant avec la lumière (18) de l'élément allongé (4) ;
un indicateur de pression (48) sur le logement (8) pour fournir un retour d'information indiquant qu'une quantité suffisante de fluide a été amenée dans l'intérieur pour dilater l'élément dilatable (6) à un état dilaté prédéterminé ; et
un piston (44) pouvant coulisser dans l'intérieur du logement (24) d'une première position vers une deuxième position lors de l'amenée du fluide dans l'intérieur (24) du logement (8), l'indicateur de pression (48) étant accouplé au piston (44) ;
l'appareil étant en outre **caractérisé en ce que** :
l'indicateur de pression comprend en outre un arbre ou un élément (48) s'étendant du piston (44) vers l'extérieur à partir de l'extrémité proximale (50) du logement (8), l'arbre ou l'élément (48) supportant un indicateur visuel (54) fournissant une indication visuelle indiquant qu'une pression et/ou un volume de fluide prédéterminés ont été introduits dans le logement (8) pour dilater l'élément dilatable (6) à l'état dilaté prédéterminé ; et
un mécanisme de détente de la pression (54) permettant l'échappement du fluide de l'intérieur du logement lors du dépassement d'une pression prédéterminée, le mécanisme de détente de la pression comprenant un orifice d'évacuation (54) communiquant avec l'intérieur du logement (24) lorsque le piston (44) se trouve au niveau de la position prédéterminée ou dans une position proximale par rapport à celle-ci, pour empêcher ainsi une dilatation excessive de l'élément dilatable (6).

2. Appareil (2) selon la revendication 1, comprenant en outre un assemblage de soupape (26) comprenant une soupape de retenue (28) permettant l'accès à l'intérieur du logement (24) lors de l'application d'une différence de pression à travers la soupape (28), l'assemblage de soupape comprenant en outre un piston plongeur mobile (30) destiné à outrepasser et à ouvrir la soupape (28) pour permettre l'accès à l'intérieur du logement (24).

3. Appareil (2) selon les revendications 1 ou 2, dans lequel l'indicateur visuel (54) englobe une série de graduations ou d'autres repères pouvant être utilisées pour déterminer le degré de dilatation de l'élément dilatable (6).

4. Appareil (2) selon l'une quelconque des revendications précédentes, dans lequel l'indicateur visuel est exposé à partir du logement (8) lorsque l'élément dilatable (6) est dilaté à l'état dilaté prédéterminé.

5. Appareil (2) selon l'une quelconque des revendications précédentes, dans lequel le piston (44) et sollicité vers la première position.

6. Appareil (2) selon l'une quelconque des revendications précédentes, comprenant en outre un élément de connexion (58) accouplé au piston (44) et s'étendant vers un emplacement distal par rapport à l'élément dilatable (6), de sorte qu'une extrémité distale (6a) de l'élément dilatable (6) est dirigée dans une direction proximale lorsque le piston (44) se déplace de la première position vers la deuxième position.

7. Appareil (2) selon la revendication 6, dans lequel le piston (44) est sollicité vers la première position, sollicitant ainsi l'élément de connexion (58) dans une direction distale pour soumettre l'élément dilatable (6) à une tension axiale et minimiser ainsi un profil de l'élément dilatable (6) dans un état contracté.

8. Appareil (2) selon la revendication 2, comprenant en outre un actionneur (34) accouplé au piston plongeur (30) pour déplacer le piston plongeur (30) entre une première position, n'outrepassant pas la soupape (28), et une deuxième position, outrepassant et ouvrant la soupape (28) et dans laquelle un orifice de fluide peut être étendu à travers la soupape (28) et mis en communication avec l'intérieur du logement (24).

9. Appareil (2) selon la revendication 8, dans lequel l'actionneur (34) peut être déplacé vers l'intérieur par rapport au logement (8) pour déplacer le piston plongeur (30) de la première position vers la deuxième position, l'actionneur (34) étant sollicité vers l'extérieur, de sorte que le piston plongeur (30) retourne vers la première position lors du relâchement de l'actionneur (34) par rapport au logement (8).

10. Appareil (2) selon les revendications 8 ou 9, dans lequel le piston plongeur (30) peut être fixé par alternance dans les première et deuxième positions, lors de l'activation et de la désactivation de l'actionneur (34).

11. Appareil (2) selon les revendications 2, 8, 9 ou 10, dans lequel la soupape (28) est une soupape en bec de canard.

12. Appareil (2) selon l'une quelconque des revendications précédentes, comprenant en outre une gaine allongée (62) comprenant des extrémités proximale et distale (64, 65) et une lumière (66) s'étendant entre elles, la lumière (66) étant dimensionnée de sorte à recevoir l'élément allongé (4) lorsque l'élément dilatable (6) se trouve dans un état contracté.

13. Appareil selon la revendication 12, comprenant en outre un composé d'étanchéité (146) pouvant être délivré à travers la gaine allongée (62) autour de l'élément allongé (4).
